# EUROPEAN PATENT SPECIFICATION

(11) **EP 2 731 960 B1**
(45) Date of publication and mention of the grant of the patent: **16.11.2016**
(21) Application number: 12731501.8
(22) Date of filing: 06.07.2012
(51) Int. Cl.: C07K 7/06, C07K 16/18, G01N 33/68

(54) **IN VITRO ASSESSMENT OF CARDIOVASCULAR EVENTS BY ASSAY FOR NEO-EPITOPES OF TITIN PROTEIN**
IN-VITRO-BEURTEILUNG KARDIOVASKULÄRER EREIGNISSE ANHAND DES TESTENS AUF NEOEPITOPE VON TITIN PROTEIN
ÉVALUATION IN VITRO D'ÉVÉNEMENTS CARDIOVASCULAIRES PAR DOSAGE DE NÉO-ÉPITOPES DE LA PROTÉINE TITINE

(30) Priority: 11.07.2011 GB 201111788
(43) Date of publication of application: 21.05.2014
(73) Proprietor: Nordic Bioscience A/S, 2730 Herlev (DK)
(72) Inventor: LEEMING, Diana Julie, DK-2450 København SV (DK); KARSDAL, Morten, DK-2100 København Ø (DK); VASSILIADIS, Efstathios, DK-2610 Rodovre (DK)
(74) Representative: Beck Greener
(86) International application number: PCT/EP2012/063278
(87) International publication number: WO 2013/007643

(56) References cited:
- M. A. M. ALI ET AL: "Titin is a Target of Matrix Metalloproteinase-2: Implications in Myocardial Ischemia/Reperfusion Injury", CIRCULATION, vol. 122, no. 20, 16 November 2010 (2010-11-16), pages 2039-2047, XP55041738, ISSN: 0009-7322, DOI: 10.1161/CIRCULATIONAHA.109.930222 cited in the application
- KARSDAL M A ET AL: "Novel combinations of Post-Translational Modification (PTM) neo-epitopes provide tissue-specific biochemical markers-are they the cause or the consequence of the disease?", CLINICAL BIOCHEMISTRY, ELSEVIER INC, US, CA, vol. 43, no. 10-11, 1 July 2010 (2010-07-01), pages 793-804, XP027085679, ISSN: 0009-9120 [retrieved on 2010-06-13]
- EFSTATHIOS VASSILIADIS ET AL: "Clinical evaluation of a matrix metalloproteinase-12 cleaved fragment of titin as a cardiovascular-specific serological biomarker", JOURNAL OF TRANSLATIONAL MEDICINE, vol. 10, no. 1, 6 July 2012 (2012-07-06), page 140, XP55041854, ISSN: 1479-5876, DOI: 10.1186/1479-5876-10-140
- VASSILIADIS ET AL: "Novel Cardiac-Specific Biomarkers and the Cardiovascular Continuum", BIOMARKER INSIGHTS, 1 January 2012 (2012-01-01), page 45, XP55041856, ISSN: 1177-2719, DOI: 10.4137/BMI.S9536

### SEQUENCE LISTING

<110> Nordic Bioscience A/S
<120> Biochemical Markers for Neurodegenerative Conditions
<130> PJS/P17675WO
<150> GB1111361.0
   <151> 2011-07-04
<160> 248
<170> BiSSAP 1.0
<210> 1
   <211> 11
   <212> PRT
   <213> Homo sapiens
<220>
   <221> SOURCE
   <222> 1..11
   <223> /mol_type="protein" /organism="Homo sapiens"
<400> 1
   Ala Ala Pro Pro Gly Gln Lys Gly Gln Ala Asn
   1               5                   10
<210> 2
   <211> 13
   <212> PRT
   <213> Homo sapiens
<220>
   <221> SOURCE
   <222> 1..13
   <223> /mol_type="protein" /organism="Homo sapiens"
<400> 2
   Ala Ala Pro Pro Gly Gln Lys Gly Gln Ala Asn Ala Thr
   1               5                   10
<210> 3
   <211> 8
   <212> PRT
   <213> Homo sapiens
<220>
   <221> SOURCE
   <222> 1..8
   <223> /mol_type="protein" /organism="Homo sapiens"
<400> 3
   Ala Pro Val Pro Met Pro Asp Leu
   1               5
<210> 4
   <211> 9
   <212> PRT
   <213> Homo sapiens
<220>
   <221> SOURCE
   <222> 1..9
   <223> /mol_type="protein" /organism="Homo sapiens"
<400> 4
   Ala Pro Val Pro Met Pro Asp Leu Lys
   1               5
<210> 5
   <211> 9
   <212> PRT
   <213> Homo sapiens
<220>
   <221> MOD_RES
   <222> 5
   <223> Oxidised Methionine
<220>
   <221> SOURCE
   <222> 1..9
   <223> /mol_type="protein" /organism="Homo sapiens"
<400> 5
   Ala Pro Val Pro Met Pro Asp Leu Lys
   1               5
<210> 6
   <211> 8
   <212> PRT
   <213> Homo sapiens
<220>
   <221> SOURCE
   <222> 1..8
   <223> /mol_type="protein" /organism="Homo sapiens"
<400> 6
   Ala Ser Leu Ala Lys Gln Gly Leu
   1               5
<210> 7
   <211> 16
   <212> PRT
   <213> Homo sapiens
<220>
   <221> SOURCE
   <222> 1..16
   <223> /mol_type="protein" /organism="Homo sapiens"
<400> 7
   Ala Thr Leu Ala Asp Glu Val Ser Ala Ser Leu Ala Lys Gln Gly Leu
   1               5                   10                  15
<210> 8
   <211> 6
   <212> PRT
   <213> Homo sapiens
<220>
   <221> SOURCE
   <222> 1..6
   <223> /mol_type="protein" /organism="Homo sapiens"
<400> 8
   Ala Thr Arg Ile Pro Ala
   1               5
<210> 9
   <211> 13
   <212> PRT
   <213> Homo sapiens
<220>
   <221> SOURCE
   <222> 1..13
   <223> /mol_type="protein" /organism="Homo sapiens"
<400> 9
   Ala Thr Arg Ile Pro Ala Lys Thr Pro Pro Ala Pro Lys
   1               5                   10
<210> 10
   <211> 23
   <212> PRT
   <213> Homo sapiens
<220>
   <221> SOURCE
   <222> 1..23
   <223> /mol_type="protein" /organism="Homo sapiens"
<400> 10
   Ala Thr Arg Ile Pro Ala Lys Thr Pro Pro Ala Pro Lys Thr Pro Pro
   1               5                   10                  15
   Ser Ser Gly Glu Pro Pro Lys
               20
<210> 11
   <211> 27
   <212> PRT
   <213> Homo sapiens
<220>
   <221> SOURCE
   <222> 1..27
   <223> /mol_type="protein" /organism="Homo sapiens"
<400> 11
   Ala Thr Arg Ile Pro Ala Lys Thr Pro Pro Ala Pro Lys Thr Pro Pro
   1               5                   10                  15
   Ser Ser Gly Glu Pro Pro Lys Ser Gly Asp Arg
               20                  25
<210> 12
   <211> 31
   <212> PRT
   <213> Homo sapiens
<220>
   <221> SOURCE
   <222> 1..31
   <223> /mol_type="protein" /organism="Homo sapiens"
<400> 12
   Ala Thr Arg Ile Pro Ala Lys Thr Pro Pro Ala Pro Lys Thr Pro Pro
   1               5                   10                  15
   Ser Ser Gly Glu Pro Pro Lys Ser Gly Asp Arg Ser Gly Tyr Ser
               20                  25                  30
<210> 13
   <211> 8
   <212> PRT
   <213> Homo sapiens
<220>
   <221> SOURCE
   <222> 1..8
   <223> /mol_type="protein" /organism="Homo sapiens"
<400> 13
   Asp Glu Ala Ala Gly His Val Thr
   1               5
<210> 14
   <211> 9
   <212> PRT
   <213> Homo sapiens
<220>
   <221> SOURCE
   <222> 1..9
   <223> /mol_type="protein" /organism="Homo sapiens"
<400> 14
   Asp Arg Lys Asp Gln Gly Gly Tyr Thr
   1               5
<210> 15
   <211> 17
   <212> PRT
   <213> Homo sapiens
<220>
   <221> SOURCE
   <222> 1..17
   <223> /mol_type="protein" /organism="Homo sapiens"
<400> 15
   Glu Ala Ala Gly His Val Thr Gln Ala Arg Met Val Ser Lys Ser Lys
   1 5 10 15
   Asp
<210> 16
   <211> 30
   <212> PRT
   <213> Homo sapiens
<220>
   <221> SOURCE
   <222> 1..30
   <223> /mol_type="protein" /organism="Homo sapiens"
<400> 16
   Glu Ala Ala Gly His Val Thr Gln Ala Arg Met Val Ser Lys Ser Lys
   1 5 10 15
   Asp Gly Thr Gly Ser Asp Asp Lys Lys Ala Lys Gly Ala Asp
   20 25 30
<210> 17
   <211> 8
   <212> PRT
   <213> Homo sapiens
<220>
   <221> SOURCE
   <222> 1..8
   <223> /mol_type="protein" /organism="Homo sapiens"
<400> 17
   Glu Asp His Ala Gly Thr Tyr Gly
   1 5
<210> 18
   <211> 9
   <212> PRT
   <213> Homo sapiens
<220>
   <221> SOURCE
   <222> 1..9
   <223> /mol_type="protein" /organism="Homo sapiens"
<400> 18
   Glu Gly Asp Thr Asp Ala Gly Leu Lys
   1 5
<210> 19
   <211> 15
   <212> PRT
   <213> Homo sapiens
<220>
   <221> SOURCE
   <222> 1..15
   <223> /mol_type="protein" /organism="Homo sapiens"
<400> 19
   Glu Asn Ala Lys Ala Lys Thr Asp His Gly Ala Glu Ile Val Tyr
   1               5                   10                  15
<210> 20
   <211> 16
   <212> PRT
   <213> Homo sapiens
<220>
   <221> SOURCE
   <222> 1..16
   <223> /mol_type="protein" /organism="Homo sapiens"
<400> 20
   Glu Asn Ala Lys Ala Lys Thr Asp His Gly Ala Glu Ile Val Tyr Lys
   1               5                   10                  15
<210> 21
   <211> 11
   <212> PRT
   <213> Homo sapiens
<220>
   <221> SOURCE
   <222> 1..11
   <223> /mol_type="protein" /organism="Homo sapiens"
<400> 21
   Glu Val Met Glu Asp His Ala Gly Thr Tyr Gly
   1               5                   10
<210> 22
   <211> 17
   <212> PRT
   <213> Homo sapiens
<220>
   <221> SOURCE
   <222> 1..17
   <223> /mol_type="protein" /organism="Homo sapiens"
<400> 22
   Glu Val Met Glu Asp His Ala Gly Thr Tyr Gly Leu Gly Asp Arg Lys
   1               5                   10                  15
   Asp
<210> 23
   <211> 17
   <212> PRT
   <213> Homo sapiens
<220>
   <221> SOURCE
   <222> 1..17
   <223> /mol_type="protein" /organism="Homo sapiens"
<220>
   <221> MOD_RES
   <222> 3
   <223> oxidised methionine
<400> 23
   Glu Val Met Glu Asp His Ala Gly Thr Tyr Gly Leu Gly Asp Arg Lys
   1               5                   10                  15
   Asp
<210> 24
   <211> 30
   <212> PRT
   <213> Homo sapiens
<220>
   <221> SOURCE
   <222> 1..30
   <223> /mol_type="protein" /organism="Homo sapiens"
<400> 24
   Glu Val Met Glu Asp His Ala Gly Thr Tyr Gly Leu Gly Asp Arg Lys
   1               5                   10                  15
   Asp Gln Gly Gly Tyr Thr Met His Gln Asp Gln Glu Gly Asp
               20                  25                  30
<210> 25
   <211> 8
   <212> PRT
   <213> Homo sapiens
<220>
   <221> SOURCE
   <222> 1..8
   <223> /mol_type="protein" /organism="Homo sapiens"
<400> 25
   Glu Val Ser Ala Ser Leu Ala Lys
   1               5
<210> 26
   <211> 11
   <212> PRT
   <213> Homo sapiens
<220>
   <221> SOURCE
   <222> 1..11
   <223> /mol_type="protein" /organism="Homo sapiens"
<400> 26
   Glu Val Ser Ala Ser Leu Ala Lys Gln Gly Leu
   1               5                   10
<210> 27
   <211> 12
   <212> PRT
   <213> Homo sapiens
<220>
   <221> SOURCE
   <222> 1..12
   <223> /mol_type="protein" /organism="Homo sapiens"
<400> 27
   Gly Ala Ala Pro Pro Gly Gln Lys Gly Gln Ala Asn
   1               5                   10
<210> 28
   <211> 14
   <212> PRT
   <213> Homo sapiens
<220>
   <221> SOURCE
   <222> 1..14
   <223> /mol_type="protein" /organism="Homo sapiens"
<400> 28
   Gly Ala Ala Pro Pro Gly Gln Lys Gly Gln Ala Asn Ala Thr
   1               5                   10
<210> 29
   <211> 13
   <212> PRT
   <213> Homo sapiens
<220>
   <221> SOURCE
   <222> 1..13
   <223> /mol_type="protein" /organism="Homo sapiens"
<400> 29
   Gly Glu Pro Pro Lys Ser Gly Asp Arg Ser Gly Tyr Ser
   1               5                   10
<210> 30
   <211> 20
   <212> PRT
   <213> Homo sapiens
<220>
   <221> SOURCE
   <222> 1..20
   <223> /mol_type="protein" /organism="Homo sapiens"
<400> 30
   Gly Ser Pro Gly Thr Pro Gly Ser Arg Ser Arg Thr Pro Ser Leu Pro
   1               5                   10                  15
   Thr Pro Pro Thr
               20
<210> 31
   <211> 18
   <212> PRT
   <213> Homo sapiens
<220>
   <221> SOURCE
   <222> 1..18
   <223> /mol_type="protein" /organism="Homo sapiens"
<400> 31
   Gly Thr Pro Gly Ser Arg Ser Arg Thr Pro Ser Leu Pro Thr Pro Pro
   1               5                   10                  15
   Thr Arg
<210> 32
   <211> 38
   <212> PRT
   <213> Homo sapiens
<220>
   <221> SOURCE
   <222> 1..38
   <223> /mol_type="protein" /organism="Homo sapiens"
<400> 32
   His Gly Ala Glu Ile Val Tyr Lys Ser Pro Val Val Ser Gly Asp Thr
   1               5                   10                  15
   Ser Pro Arg His Leu Ser Asn Val Ser Ser Thr Gly Ser Ile Asp Met
               20                  25                  30
   Val Asp Ser Pro Gln Leu
           35
<210> 33
   <211> 11
   <212> PRT
   <213> Homo sapiens
<220>
   <221> SOURCE
   <222> 1..11
   <223> /mol_type="protein" /organism="Homo sapiens"
<400> 33
   His Lys Pro Gly Gly Gly Gln Val Glu Val Lys
   1 5 10
<210> 34
   <211> 11
   <212> PRT
   <213> Homo sapiens
<220>
   <221> SOURCE
   <222> 1..11
   <223> /mol_type="protein" /organism="Homo sapiens"
<400> 34
   His Val Pro Gly Gly Gly Asn Lys Lys Ile Glu
   1 5 10
<210> 35
   <211> 12
   <212> PRT
   <213> Homo sapiens
<220>
   <221> SOURCE
   <222> 1..12
   <223> /mol_type="protein" /organism="Homo sapiens"
<400> 35
   His Val Pro Gly Gly Gly Asn Lys Lys Ile Glu Thr
   1 5 10
<210> 36
   <211> 9
   <212> PRT
   <213> Homo sapiens
<220>
   <221> SOURCE
   <222> 1..9
   <223> /mol_type="protein" /organism="Homo sapiens"
<400> 36
   His Val Pro Gly Gly Gly Ser Val Gln
   1 5
<210> 37
   <211> 10
   <212> PRT
   <213> Homo sapiens
<220>
   <221> SOURCE
   <222> 1..10
   <223> /mol_type="protein" /organism="Homo sapiens"
<400> 37
   Ile Pro Ala Lys Thr Pro Pro Ala Pro Lys
   1               5                   10
<210> 38
   <211> 24
   <212> PRT
   <213> Homo sapiens
<220>
   <221> SOURCE
   <222> 1..24
   <223> /mol_type="protein" /organism="Homo sapiens"
<400> 38
   Ile Pro Ala Lys Thr Pro Pro Ala Pro Lys Thr Pro Pro Ser Ser Gly
   1               5                   10                  15
   Glu Pro Pro Lys Ser Gly Asp Arg
               20
<210> 39
   <211> 28
   <212> PRT
   <213> Homo sapiens
<220>
   <221> SOURCE
   <222> 1..28
   <223> /mol_type="protein" /organism="Homo sapiens"
<400> 39
   Ile Pro Ala Lys Thr Pro Pro Ala Pro Lys Thr Pro Pro Ser Ser Gly
   1               5                   10                  15
   Glu Pro Pro Lys Ser Gly Asp Arg Ser Gly Tyr Ser
               20                  25
<210> 40
   <211> 13
   <212> PRT
   <213> Homo sapiens
<220>
   <221> SOURCE
   <222> 1..13
   <223> /mol_type="protein" /organism="Homo sapiens"
<400> 40
   Lys Ala Lys Thr Asp His Gly Ala Glu Ile Val Tyr Lys
   1               5                   10
<210> 41
   <211> 23
   <212> PRT
   <213> Homo sapiens
<220>
   <221> SOURCE
   <222> 1..23
   <223> /mol_type="protein" /organism="Homo sapiens"
<400> 41
   Lys Ser Lys Asp Gly Thr Gly Ser Asp Asp Lys Lys Ala Lys Gly Ala
   1               5                   10                  15
   Asp Gly Lys Thr Lys Ile Ala
               20
<210> 42
   <211> 15
   <212> PRT
   <213> Homo sapiens
<220>
   <221> SOURCE
   <222> 1..15
   <223> /mol_type="protein" /organism="Homo sapiens"
<400> 42
   Lys Ser Pro Val Val Ser Gly Asp Thr Ser Pro Arg His Leu Ser
   1               5                   10                  15
<210> 43
   <211> 17
   <212> PRT
   <213> Homo sapiens
<220>
   <221> SOURCE
   <222> 1..17
   <223> /mol_type="protein" /organism="Homo sapiens"
<400> 43
   Lys Thr Pro Pro Ala Pro Lys Thr Pro Pro Ser Ser Gly Glu Pro Pro
   1               5                   10                  15
   Lys
<210> 44
   <211> 21
   <212> PRT
   <213> Homo sapiens
<220>
   <221> SOURCE
   <222> 1..21
   <223> /mol_type="protein" /organism="Homo sapiens"
<400> 44
   Lys Thr Pro Pro Ala Pro Lys Thr Pro Pro Ser Ser Gly Glu Pro Pro
   1               5                   10                  15
   Lys Ser Gly Asp Arg
               20
<210> 45
   <211> 25
   <212> PRT
   <213> Homo sapiens
<220>
   <221> SOURCE
   <222> 1..25
   <223> /mol_type="protein" /organism="Homo sapiens"
<400> 45
   Lys Thr Pro Pro Ala Pro Lys Thr Pro Pro Ser Ser Gly Glu Pro Pro
   1               5                   10                  15
   Lys Ser Gly Asp Arg Ser Gly Tyr Ser
               20                  25
<210> 46
   <211> 6
   <212> PRT
   <213> Homo sapiens
<220>
   <221> SOURCE
   <222> 1..6
   <223> /mol_type="protein" /organism="Homo sapiens"
<400> 46
   Leu Ala Lys Gln Gly Leu
   1               5
<210> 47
   <211> 17
   <212> PRT
   <213> Homo sapiens
<220>
   <221> SOURCE
   <222> 1..17
   <223> /mol_type="protein" /organism="Homo sapiens"
<400> 47
   Leu Ala Thr Leu Ala Asp Glu Val Ser Ala Ser Leu Ala Lys Gln Gly
   1               5                   10                  15
   Leu
<210> 48
   <211> 31
   <212> PRT
   <213> Homo sapiens
<220>
   <221> SOURCE
   <222> 1..31
   <223> /mol_type="protein" /organism="Homo sapiens"
<400> 48
   Leu Lys Asn Val Lys Ser Lys Ile Gly Ser Thr Glu Asn Leu Lys His
   1               5                   10                  15
   Gln Pro Gly Gly Gly Lys Val Gln Ile Ile Asn Lys Lys Leu Asp
               20                  25                  30
<210> 49
   <211> 38
   <212> PRT
   <213> Homo sapiens
<220>
   <221> SOURCE
   <222> 1..38
   <223> /mol_type="protein" /organism="Homo sapiens"
<400> 49
   Leu Lys Asn Val Lys Ser Lys Ile Gly Ser Thr Glu Asn Leu Lys His
   1               5                   10                  15
   Gln Pro Gly Gly Gly Lys Val Gln Ile Ile Asn Lys Lys Leu Asp Leu
               20                  25                  30
   Ser Asn Val Gln Ser Lys
           35
<210> 50
   <211> 7
   <212> PRT
   <213> Homo sapiens
<220>
   <221> SOURCE
   <222> 1..7
   <223> /mol_type="protein" /organism="Homo sapiens"
<400> 50
   Leu Pro Thr Pro Pro Thr Arg
   1               5
<210> 51
   <211> 13
   <212> PRT
   <213> Homo sapiens
<220>
   <221> SOURCE
   <222> 1..13
   <223> /mol_type="protein" /organism="Homo sapiens"
<400> 51
   Leu Pro Thr Pro Pro Thr Arg Glu Pro Lys Lys Val Ala
   1               5                   10
<210> 52
   <211> 15
   <212> PRT
   <213> Homo sapiens
<220>
   <221> SOURCE
   <222> 1..15
   <223> /mol_type="protein" /organism="Homo sapiens"
<400> 52
   Leu Pro Thr Pro Pro Thr Arg Glu Pro Lys Lys Val Ala Val Val
   1               5                   10                  15
<210> 53
   <211> 14
   <212> PRT
   <213> Homo sapiens
<220>
   <221> SOURCE
   <222> 1..14
   <223> /mol_type="protein" /organism="Homo sapiens"
<400> 53
   Met His Gln Asp Gln Glu Gly Asp Thr Asp Ala Gly Leu Lys
   1               5                   10
<210> 54
   <211> 14
   <212> PRT
   <213> Homo sapiens
<220>
   <221> SOURCE
   <222> 1..14
   <223> /mol_type="protein" /organism="Homo sapiens"
<220>
   <221> MOD_RES
   <222> 1
   <223> oxidised methionine
<400> 54
   Met His Gln Asp Gln Glu Gly Asp Thr Asp Ala Gly Leu Lys
   1 5 10
<210> 55
   <211> 23
   <212> PRT
   <213> Homo sapiens
<220>
   <221> SOURCE
   <222> 1..23
   <223> /mol_type="protein" /organism="Homo sapiens"
<400> 55
   Met Val Asp Ser Pro Gln Leu Ala Thr Leu Ala Asp Glu Val Ser Ala
   1               5                   10                  15
   Ser Leu Ala Lys Gln Gly Leu
               20
<210> 56
   <211> 32
   <212> PRT
   <213> Homo sapiens
<220>
   <221> SOURCE
   <222> 1..32
   <223> /mol_type="protein" /organism="Homo sapiens"
<400> 56
   Asn Ala Thr Arg Ile Pro Ala Lys Thr Pro Pro Ala Pro Lys Thr Pro
   1               5                   10                  15
   Pro Ser Ser Gly Glu Pro Pro Lys Ser Gly Asp Arg Ser Gly Tyr Ser
               20                  25                  30
<210> 57
   <211> 12
   <212> PRT
   <213> Homo sapiens
<220>
   <221> SOURCE
   <222> 1..12
   <223> /mol_type="protein" /organism="Homo sapiens"
<400> 57
   Asn Ile His His Lys Pro Gly Gly Gly Gln Val Glu
   1               5                   10
<210> 58
   <211> 14
   <212> PRT
   <213> Homo sapiens
<220>
   <221> SOURCE
   <222> 1..14
   <223> /mol_type="protein" /organism="Homo sapiens"
<400> 58
   Asn Ile His His Lys Pro Gly Gly Gly Gln Val Glu Val Lys
   1 5 10
<210> 59
   <211> 15
   <212> PRT
   <213> Homo sapiens
<220>
   <221> SOURCE
   <222> 1..15
   <223> /mol_type="protein" /organism="Homo sapiens"
<400> 59
   Pro Gly Ser Pro Gly Thr Pro Gly Ser Arg Ser Arg Thr Pro Ser
   1 5 10 15
<210> 60
   <211> 6
   <212> PRT
   <213> Homo sapiens
<220>
   <221> SOURCE
   <222> 1..6
   <223> /mol_type="protein" /organism="Homo sapiens"
<400> 60
   Pro Met Pro Asp Leu Lys
   1 5
<210> 61
   <211> 23
   <212> PRT
   <213> Homo sapiens
<220>
   <221> SOURCE
   <222> 1..23
   <223> /mol_type="protein" /organism="Homo sapiens"
<400> 61
   Pro Pro Ala Pro Lys Thr Pro Pro Ser Ser Gly Glu Pro Pro Lys Ser
   1 5 10 15
   Gly Asp Arg Ser Gly Tyr Ser
   20
<210> 62
   <211> 11
   <212> PRT
   <213> Homo sapiens
<220>
   <221> SOURCE
   <222> 1..11
   <223> /mol_type="protein" /organism="Homo sapiens"
<400> 62
   Pro Pro Lys Ser Gly Asp Arg Ser Gly Tyr Ser
   1               5                   10
<210> 63
   <211> 10
   <212> PRT
   <213> Homo sapiens
<220>
   <221> SOURCE
   <222> 1..10
   <223> /mol_type="protein" /organism="Homo sapiens"
<400> 63
   Pro Pro Thr Arg Glu Pro Lys Lys Val Ala
   1               5                   10
<210> 64
   <211> 16
   <212> PRT
   <213> Homo sapiens
<220>
   <221> SOURCE
   <222> 1..16
   <223> /mol_type="protein" /organism="Homo sapiens"
<400> 64
   Pro Ser Ser Gly Glu Pro Pro Lys Ser Gly Asp Arg Ser Gly Tyr Ser
   1               5                   10                  15
<210> 65
   <211> 8
   <212> PRT
   <213> Homo sapiens
<220>
   <221> SOURCE
   <222> 1..8
   <223> /mol_type="protein" /organism="Homo sapiens"
<400> 65
   Pro Val Pro Met Pro Asp Leu Lys
   1 5
<210> 66
   <211> 8
   <212> PRT
   <213> Homo sapiens
<220>
   <221> SOURCE
   <222> 1..8
   <223> /mol_type="protein" /organism="Homo sapiens"
<220>
   <221> MOD_RES
   <222> 4
   <223> oxidised methionine
<400> 66
   Pro Val Pro Met Pro Asp Leu Lys
   1 5
<210> 67
   <211> 6
   <212> PRT
   <213> Homo sapiens
<220>
   <221> SOURCE
   <222> 1..6
   <223> /mol_type="protein" /organism="Homo sapiens"
<400> 67
   Gln Ala Arg Met Val Ser
   1 5
<210> 68
   <211> 18
   <212> PRT
   <213> Homo sapiens
<220>
   <221> SOURCE
   <222> 1..18
   <223> /mol_type="protein" /organism="Homo sapiens"
<400> 68
   Gln Leu Ala Thr Leu Ala Asp Glu Val Ser Ala Ser Leu Ala Lys Gln
   1 5 10 15
   Gly Leu
<210> 69
   <211> 10
   <212> PRT
   <213> Homo sapiens
<220>
   <221> SOURCE
   <222> 1..10
   <223> /mol_type="protein" /organism="Homo sapiens"
<400> 69
   Gln Thr Ala Pro Val Pro Met Pro Asp Leu
   1               5                   10
<210> 70
   <211> 11
   <212> PRT
   <213> Homo sapiens
<220>
   <221> SOURCE
   <222> 1..11
   <223> /mol_type="protein" /organism="Homo sapiens"
<400> 70
   Gln Thr Ala Pro Val Pro Met Pro Asp Leu Lys
   1               5                   10
<210> 71
   <211> 11
   <212> PRT
   <213> Homo sapiens
<220>
   <221> SOURCE
   <222> 1..11
   <223> /mol_type="protein" /organism="Homo sapiens"
<220>
   <221> MOD_RES
   <222> 7
   <223> oxidised methionine
<400> 71
   Gln Thr Ala Pro Val Pro Met Pro Asp Leu Lys
   1               5                   10
<210> 72
   <211> 17
   <212> PRT
   <213> Homo sapiens
<220>
   <221> SOURCE
   <222> 1..17
   <223> /mol_type="protein" /organism="Homo sapiens"
<400> 72
   Arg Glu Asn Ala Lys Ala Lys Thr Asp His Gly Ala Glu Ile Val Tyr
   1               5                   10                  15
   Lys
<210> 73
   <211> 11
   <212> PRT
   <213> Homo sapiens
<220>
   <221> SOURCE
   <222> 1..11
   <223> /mol_type="protein" /organism="Homo sapiens"
<400> 73
   Arg Ile Pro Ala Lys Thr Pro Pro Ala Pro Lys
   1               5                   10
<210> 74
   <211> 15
   <212> PRT
   <213> Homo sapiens
<220>
   <221> SOURCE
   <222> 1..15
   <223> /mol_type="protein" /organism="Homo sapiens"
<400> 74
   Arg Ile Pro Ala Lys Thr Pro Pro Ala Pro Lys Thr Pro Pro Ser
   1               5                   10                  15
<210> 75
   <211> 21
   <212> PRT
   <213> Homo sapiens
<220>
   <221> SOURCE
   <222> 1..21
   <223> /mol_type="protein" /organism="Homo sapiens"
<400> 75
   Arg Ile Pro Ala Lys Thr Pro Pro Ala Pro Lys Thr Pro Pro Ser Ser
   1               5                   10                  15
   Gly Glu Pro Pro Lys
               20
<210> 76
   <211> 25
   <212> PRT
   <213> Homo sapiens
<220>
   <221> SOURCE
   <222> 1..25
   <223> /mol_type="protein" /organism="Homo sapiens"
<400> 76
   Arg Ile Pro Ala Lys Thr Pro Pro Ala Pro Lys Thr Pro Pro Ser Ser
   1               5                   10                  15
   Gly Glu Pro Pro Lys Ser Gly Asp Arg
               20                  25
<210> 77
   <211> 29
   <212> PRT
   <213> Homo sapiens
<220>
   <221> SOURCE
   <222> 1..29
   <223> /mol_type="protein" /organism="Homo sapiens"
<400> 77
   Arg Ile Pro Ala Lys Thr Pro Pro Ala Pro Lys Thr Pro Pro Ser Ser
   1               5                   10                  15
   Gly Glu Pro Pro Lys Ser Gly Asp Arg Ser Gly Tyr Ser
               20                  25
<210> 78
   <211> 12
   <212> PRT
   <213> Homo sapiens
<220>
   <221> SOURCE
   <222> 1..12
   <223> /mol_type="protein" /organism="Homo sapiens"
<400> 78
   Arg Lys Asp Gln Gly Gly Tyr Thr Met His Gln Asp
   1               5                   10
<210> 79
   <211> 18
   <212> PRT
   <213> Homo sapiens
<220>
   <221> SOURCE
   <222> 1..18
   <223> /mol_type="protein" /organism="Homo sapiens"
<400> 79
   Arg Lys Asp Gln Gly Gly Tyr Thr Met His Gln Asp Gln Glu Gly Asp
   1               5                   10                  15
   Thr Asp
<210> 80
   <211> 10
   <212> PRT
   <213> Homo sapiens
<220>
   <221> SOURCE
   <222> 1..10
   <223> /mol_type="protein" /organism="Homo sapiens"
<400> 80
   Arg Thr Pro Pro Lys Ser Pro Ser Ser Ala
   1               5                   10
<210> 81
   <211> 14
   <212> PRT
   <213> Homo sapiens
<220>
   <221> SOURCE
   <222> 1..14
   <223> /mol_type="protein" /organism="Homo sapiens"
<400> 81
   Arg Thr Pro Pro Lys Ser Pro Ser Ser Ala Lys Ser Arg Leu
   1               5                   10
<210> 82
   <211> 15
   <212> PRT
   <213> Homo sapiens
<220>
   <221> SOURCE
   <222> 1..15
   <223> /mol_type="protein" /organism="Homo sapiens"
<400> 82
   Arg Thr Pro Pro Lys Ser Pro Ser Ser Ala Lys Ser Arg Leu Gln
   1               5                   10                  15
<210> 83
   <211> 10
   <212> PRT
   <213> Homo sapiens
<220>
   <221> SOURCE
   <222> 1..10
   <223> /mol_type="protein" /organism="Homo sapiens"
<400> 83
   Arg Thr Pro Ser Leu Pro Thr Pro Pro Thr
   1               5                   10
<210> 84
   <211> 11
   <212> PRT
   <213> Homo sapiens
<220>
   <221> SOURCE
   <222> 1..11
   <223> /mol_type="protein" /organism="Homo sapiens"
<400> 84
   Arg Thr Pro Ser Leu Pro Thr Pro Pro Thr Arg
   1               5                   10
<210> 85
   <211> 14
   <212> PRT
   <213> Homo sapiens
<220>
   <221> SOURCE
   <222> 1..14
   <223> /mol_type="protein" /organism="Homo sapiens"
<400> 85
   Arg Thr Pro Ser Leu Pro Thr Pro Pro Thr Arg Glu Pro Lys
   1               5                   10
<210> 86
   <211> 17
   <212> PRT
   <213> Homo sapiens
<220>
   <221> SOURCE
   <222> 1..17
   <223> /mol_type="protein" /organism="Homo sapiens"
<400> 86
   Arg Thr Pro Ser Leu Pro Thr Pro Pro Thr Arg Glu Pro Lys Lys Val
   1               5                   10                  15
   Ala
<210> 87
   <211> 9
   <212> PRT
   <213> Homo sapiens
<220>
   <221> SOURCE
   <222> 1..9
   <223> /mol_type="protein" /organism="Homo sapiens"
<400> 87
   Ser Ala Ser Leu Ala Lys Gln Gly Leu
   1               5
<210> 88
   <211> 6
   <212> PRT
   <213> Homo sapiens
<220>
   <221> SOURCE
   <222> 1..6
   <223> /mol_type="protein" /organism="Homo sapiens"
<400> 88
   Ser Glu Lys Leu Asp Phe
   1               5
<210> 89
   <211> 10
   <212> PRT
   <213> Homo sapiens
<220>
   <221> SOURCE
   <222> 1..10
   <223> /mol_type="protein" /organism="Homo sapiens"
<400> 89
   Ser Gly Asp Thr Ser Pro Arg His Leu Ser
   1               5                   10
<210> 90
   <211> 14
   <212> PRT
   <213> Homo sapiens
<220>
   <221> SOURCE
   <222> 1..14
   <223> /mol_type="protein" /organism="Homo sapiens"
<400> 90
   Ser Gly Glu Pro Pro Lys Ser Gly Asp Arg Ser Gly Tyr Ser
   1               5                   10
<210> 91
   <211> 7
   <212> PRT
   <213> Homo sapiens
<220>
   <221> SOURCE
   <222> 1..7
   <223> /mol_type="protein" /organism="Homo sapiens"
<400> 91
   Ser Leu Ala Lys Gln Gly Leu
   1               5
<210> 92
   <211> 11
   <212> PRT
   <213> Homo sapiens
<220>
   <221> SOURCE
   <222> 1..11
   <223> /mol_type="protein" /organism="Homo sapiens"
<400> 92
   Ser Pro Gly Ser Pro Gly Thr Pro Gly Ser Arg
   1               5                   10
<210> 93
   <211> 12
   <212> PRT
   <213> Homo sapiens
<220>
   <221> SOURCE
   <222> 1..12
   <223> /mol_type="protein" /organism="Homo sapiens"
<400> 93
   Ser Pro Gly Ser Pro Gly Thr Pro Gly Ser Arg Ser
   1               5                   10
<210> 94
   <211> 13
   <212> PRT
   <213> Homo sapiens
<220>
   <221> SOURCE
   <222> 1..13
   <223> /mol_type="protein" /organism="Homo sapiens"
<400> 94
   Ser Pro Gly Ser Pro Gly Thr Pro Gly Ser Arg Ser Arg
   1 5 10
<210> 95
   <211> 14
   <212> PRT
   <213> Homo sapiens
<220>
   <221> SOURCE
   <222> 1..14
   <223> /mol_type="protein" /organism="Homo sapiens"
<400> 95
   Ser Pro Gly Ser Pro Gly Thr Pro Gly Ser Arg Ser Arg Thr
   1 5 10
<210> 96
   <211> 16
   <212> PRT
   <213> Homo sapiens
<220>
   <221> SOURCE
   <222> 1..16
   <223> /mol_type="protein" /organism="Homo sapiens"
<400> 96
   Ser Pro Gly Ser Pro Gly Thr Pro Gly Ser Arg Ser Arg Thr Pro Ser
   1 5 10 15
<210> 97
   <211> 22
   <212> PRT
   <213> Homo sapiens
<220>
   <221> SOURCE
   <222> 1..22
   <223> /mol_type="protein" /organism="Homo sapiens"
<400> 97
   Ser Pro Gly Ser Pro Gly Thr Pro Gly Ser Arg Ser Arg Thr Pro Ser
   1               5                   10                  15
   Leu Pro Thr Pro Pro Thr
               20
<210> 98
   <211> 23
   <212> PRT
   <213> Homo sapiens
<220>
   <221> SOURCE
   <222> 1..23
   <223> /mol_type="protein" /organism="Homo sapiens"
<400> 98
   Ser Pro Gly Ser Pro Gly Thr Pro Gly Ser Arg Ser Arg Thr Pro Ser
   1               5                   10                  15
   Leu Pro Thr Pro Pro Thr Arg
               20
<210> 99
   <211> 9
   <212> PRT
   <213> Homo sapiens
<220>
   <221> SOURCE
   <222> 1..9
   <223> /mol_type="protein" /organism="Homo sapiens"
<400> 99
   Ser Pro Gly Thr Pro Gly Ser Arg Ser
   1               5
<210> 100
   <211> 26
   <212> PRT
   <213> Homo sapiens
<220>
   <221> SOURCE
   <222> 1..26
   <223> /mol_type="protein" /organism="Homo sapiens"
<400> 100
   Ser Pro Gly Thr Pro Gly Ser Arg Ser Arg Thr Pro Ser Leu Pro Thr
   1               5                   10                  15
   Pro Pro Thr Arg Glu Pro Lys Lys Val Ala
               20                  25
<210> 101
   <211> 20
   <212> PRT
   <213> Homo sapiens
<220>
   <221> SOURCE
   <222> 1..20
   <223> /mol_type="protein" /organism="Homo sapiens"
<400> 101
   Ser Pro Gln Leu Ala Thr Leu Ala Asp Glu Val Ser Ala Ser Leu Ala
   1               5                   10                  15
   Lys Gln Gly Leu
               20
<210> 102
   <211> 6
   <212> PRT
   <213> Homo sapiens
<220>
   <221> SOURCE
   <222> 1..6
   <223> /mol_type="protein" /organism="Homo sapiens"
<400> 102
   Ser Pro Arg His Leu Ser
   1               5
<210> 103
   <211> 9
   <212> PRT
   <213> Homo sapiens
<220>
   <221> SOURCE
   <222> 1..9
   <223> /mol_type="protein" /organism="Homo sapiens"
<400> 103
   Ser Pro Ser Ser Ala Lys Ser Arg Leu
   1               5
<210> 104
   <211> 10
   <212> PRT
   <213> Homo sapiens
<220>
   <221> SOURCE
   <222> 1..10
   <223> /mol_type="protein" /organism="Homo sapiens"
<400> 104
   Ser Pro Ser Ser Ala Lys Ser Arg Leu Gln
   1 5 10
<210> 105
   <211> 14
   <212> PRT
   <213> Homo sapiens
<220>
   <221> SOURCE
   <222> 1..14
   <223> /mol_type="protein" /organism="Homo sapiens"
<400> 105
   Ser Pro Val Val Ser Gly Asp Thr Ser Pro Arg His Leu Ser
   1 5 10
<210> 106
   <211> 14
   <212> PRT
   <213> Homo sapiens
<220>
   <221> SOURCE
   <222> 1..14
   <223> /mol_type="protein" /organism="Homo sapiens"
<400> 106
   Ser Arg Ser Arg Thr Pro Ser Leu Pro Thr Pro Pro Thr Arg
   1 5 10
<210> 107
   <211> 11
   <212> PRT
   <213> Homo sapiens
<220>
   <221> SOURCE
   <222> 1..11
   <223> /mol_type="protein" /organism="Homo sapiens"
<400> 107
   Ser Arg Thr Pro Ser Leu Pro Thr Pro Pro Thr
   1 5 10
<210> 108
   <211> 15
   <212> PRT
   <213> Homo sapiens
<220>
   <221> SOURCE
   <222> 1..15
   <223> /mol_type="protein" /organism="Homo sapiens"
<400> 108
   Ser Arg Thr Pro Ser Leu Pro Thr Pro Pro Thr Arg Glu Pro Lys
   1 5 10 15
<210> 109
   <211> 18
   <212> PRT
   <213> Homo sapiens
<220>
   <221> SOURCE
   <222> 1..18
   <223> /mol_type="protein" /organism="Homo sapiens"
<400> 109
   Ser Arg Thr Pro Ser Leu Pro Thr Pro Pro Thr Arg Glu Pro Lys Lys
   1 5 10 15
   Val Ala
<210> 110
   <211> 6
   <212> PRT
   <213> Homo sapiens
<220>
   <221> SOURCE
   <222> 1..6
   <223> /mol_type="protein" /organism="Homo sapiens"
<400> 110
   Ser Thr Glu Asn Leu Lys
   1 5
<210> 111
   <211> 9
   <212> PRT
   <213> Homo sapiens
<220>
   <221> SOURCE
   <222> 1..9
   <223> /mol_type="protein" /organism="Homo sapiens"
<400> 111
   Thr Ala Pro Val Pro Met Pro Asp Leu
   1 5
<210> 112
   <211> 10
   <212> PRT
   <213> Homo sapiens
<220>
   <221> SOURCE
   <222> 1..10
   <223> /mol_type="protein" /organism="Homo sapiens"
<400> 112
   Thr Ala Pro Val Pro Met Pro Asp Leu Lys
   1               5                   10
<210> 113
   <211> 10
   <212> PRT
   <213> Homo sapiens
<220>
   <221> SOURCE
   <222> 1..10
   <223> /mol_type="protein" /organism="Homo sapiens"
<220>
   <221> MOD_RES
   <222> 6
   <223> oxidised methionine
<400> 113
   Thr Ala Pro Val Pro Met Pro Asp Leu Lys
   1               5                   10
<210> 114
   <211> 11
   <212> PRT
   <213> Homo sapiens
<220>
   <221> SOURCE
   <222> 1..11
   <223> /mol_type="protein" /organism="Homo sapiens"
<400> 114
   Thr Ala Pro Val Pro Met Pro Asp Leu Lys Asn
   1               5                   10
<210> 115
   <211> 13
   <212> PRT
   <213> Homo sapiens
<220>
   <221> SOURCE
   <222> 1..13
   <223> /mol_type="protein" /organism="Homo sapiens"
<400> 115
   Thr Ala Pro Val Pro Met Pro Asp Leu Lys Asn Val Lys
   1 5 10
<210> 116
   <211> 15
   <212> PRT
   <213> Homo sapiens
<220>
   <221> SOURCE
   <222> 1..15
   <223> /mol_type="protein" /organism="Homo sapiens"
<400> 116
   Thr Leu Ala Asp Glu Val Ser Ala Ser Leu Ala Lys Gln Gly Leu
   1 5 10 15
<210> 117
   <211> 6
   <212> PRT
   <213> Homo sapiens
<220>
   <221> SOURCE
   <222> 1..6
   <223> /mol_type="protein" /organism="Homo sapiens"
<400> 117
   Thr Pro Pro Ala Pro Lys
   1 5
<210> 118
   <211> 16
   <212> PRT
   <213> Homo sapiens
<220>
   <221> SOURCE
   <222> 1..16
   <223> /mol_type="protein" /organism="Homo sapiens"
<400> 118
   Thr Pro Pro Ala Pro Lys Thr Pro Pro Ser Ser Gly Glu Pro Pro Lys
   1 5 10 15
<210> 119
   <211> 20
   <212> PRT
   <213> Homo sapiens
<220>
   <221> SOURCE
   <222> 1..20
   <223> /mol_type="protein" /organism="Homo sapiens"
<400> 119
   Thr Pro Pro Ala Pro Lys Thr Pro Pro Ser Ser Gly Glu Pro Pro Lys
   1               5                   10                  15
   Ser Gly Asp Arg
               20
<210> 120
   <211> 24
   <212> PRT
   <213> Homo sapiens
<220>
   <221> SOURCE
   <222> 1..24
   <223> /mol_type="protein" /organism="Homo sapiens"
<400> 120
   Thr Pro Pro Ala Pro Lys Thr Pro Pro Ser Ser Gly Glu Pro Pro Lys
   1               5                   10                  15
   Ser Gly Asp Arg Ser Gly Tyr Ser
               20
<210> 121
   <211> 10
   <212> PRT
   <213> Homo sapiens
<220>
   <221> SOURCE
   <222> 1..10
   <223> /mol_type="protein" /organism="Homo sapiens"
<400> 121
   Thr Pro Pro Lys Ser Pro Ser Ser Ala Lys
   1               5                   10
<210> 122
   <211> 13
   <212> PRT
   <213> Homo sapiens
<220>
   <221> SOURCE
   <222> 1..13
   <223> /mol_type="protein" /organism="Homo sapiens"
<400> 122
   Thr Pro Pro Lys Ser Pro Ser Ser Ala Lys Ser Arg Leu
   1               5                   10
<210> 123
   <211> 14
   <212> PRT
   <213> Homo sapiens
<220>
   <221> SOURCE
   <222> 1..14
   <223> /mol_type="protein" /organism="Homo sapiens"
<400> 123
   Thr Pro Pro Lys Ser Pro Ser Ser Ala Lys Ser Arg Leu Gln
   1               5                   10
<210> 124
   <211> 14
   <212> PRT
   <213> Homo sapiens
<220>
   <221> SOURCE
   <222> 1..14
   <223> /mol_type="protein" /organism="Homo sapiens"
<400> 124
   Thr Pro Pro Ser Ser Gly Glu Pro Pro Lys Ser Gly Asp Arg
   1               5                   10
<210> 125
   <211> 18
   <212> PRT
   <213> Homo sapiens
<220>
   <221> SOURCE
   <222> 1..18
   <223> /mol_type="protein" /organism="Homo sapiens"
<400> 125
   Thr Pro Pro Ser Ser Gly Glu Pro Pro Lys Ser Gly Asp Arg Ser Gly
   1               5                   10                  15
   Tyr Ser
<210> 126
   <211> 11
   <212> PRT
   <213> Homo sapiens
<220>
   <221> SOURCE
   <222> 1..11
   <223> /mol_type="protein" /organism="Homo sapiens"
<400> 126
   Thr Pro Arg Gly Ala Ala Pro Pro Gly Gln Lys
   1 5 10
<210> 127
   <211> 15
   <212> PRT
   <213> Homo sapiens
<220>
   <221> SOURCE
   <222> 1..15
   <223> /mol_type="protein" /organism="Homo sapiens"
<400> 127
   Thr Pro Arg Gly Ala Ala Pro Pro Gly Gln Lys Gly Gln Ala Asn
   1 5 10 15
<210> 128
   <211> 17
   <212> PRT
   <213> Homo sapiens
<220>
   <221> SOURCE
   <222> 1..17
   <223> /mol_type="protein" /organism="Homo sapiens"
<400> 128
   Thr Pro Arg Gly Ala Ala Pro Pro Gly Gln Lys Gly Gln Ala Asn Ala
   1 5 10 15
   Thr
<210> 129
   <211> 23
   <212> PRT
   <213> Homo sapiens
<220>
   <221> SOURCE
   <222> 1..23
   <223> /mol_type="protein" /organism="Homo sapiens"
<400> 129
   Thr Pro Ser Leu Glu Asp Glu Ala Ala Gly His Val Thr Gln Ala Arg
   1 5 10 15
   Met Val Ser Lys Ser Lys Asp
   20
<210> 130
   <211> 30
   <212> PRT
   <213> Homo sapiens
<220>
   <221> SOURCE
   <222> 1..30
   <223> /mol_type="protein" /organism="Homo sapiens"
<400> 130
   Thr Arg Ile Pro Ala Lys Thr Pro Pro Ala Pro Lys Thr Pro Pro Ser
   1               5                   10                  15
   Ser Gly Glu Pro Pro Lys Ser Gly Asp Arg Ser Gly Tyr Ser
               20                   25                  30
<210> 131
   <211> 16
   <212> PRT
   <213> Homo sapiens
<220>
   <221> SOURCE
   <222> 1..16
   <223> /mol_type="protein" /organism="Homo sapiens"
<400> 131
   Thr Ser Pro Arg His Leu Ser Asn Val Ser Ser Thr Gly Ser Ile Asp
   1               5                   10                  15
<210> 132
   <211> 23
   <212> PRT
   <213> Homo sapiens
<220>
   <221> SOURCE
   <222> 1..23
   <223> /mol_type="protein" /organism="Homo sapiens"
<400> 132
   Thr Ser Pro Arg His Leu Ser Asn Val Ser Ser Thr Gly Ser Ile Asp
   1               5                   10                  15
   Met Val Asp Ser Pro Gln Leu
               20
<210> 133
   <211> 39
   <212> PRT
   <213> Homo sapiens
<220>
   <221> SOURCE
   <222> 1..39
   <223> /mol_type="protein" /organism="Homo sapiens"
<220>
   <221> MOD_RES
   <222> 17
   <223> oxidised methionine
<400> 133
   Thr Ser Pro Arg His Leu Ser Asn Val Ser Ser Thr Gly Ser Ile Asp
   1               5                   10                  15
   Met Val Asp Ser Pro Gln Leu Ala Thr Leu Ala Asp Glu Val Ser Ala
               20                  25                  30
   Ser Leu Ala Lys Gln Gly Leu
           35
<210> 134
   <211> 10
   <212> PRT
   <213> Homo sapiens
<220>
   <221> SOURCE
   <222> 1..10
   <223> /mol_type="protein" /organism="Homo sapiens"
<400> 134
   Val Pro Gly Gly Gly Asn Lys Lys Ile Glu
   1               5                   10
<210> 135
   <211> 10
   <212> PRT
   <213> Homo sapiens
<220>
   <221> SOURCE
   <222> 1..10
   <223> /mol_type="protein" /organism="Homo sapiens"
<400> 135
   Val Ser Ala Ser Leu Ala Lys Gln Gly Leu
   1               5                   10
<210> 136
   <211> 6
   <212> PRT
   <213> Homo sapiens
<220>
   <221> SOURCE
   <222> 1..6
   <223> /mol_type="protein" /organism="Homo sapiens"
<400> 136
   Ala Ala Pro Pro Gly Gln
   1               5
<210> 137
   <211> 6
   <212> PRT
   <213> Homo sapiens
<220>
   <221> SOURCE
   <222> 1..6
   <223> /mol_type="protein" /organism="Homo sapiens"
<400> 137
   Ala Pro Val Pro Met Pro
   1 5
<210> 138
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 5
   <223> oxidised methionine
<400> 138
   Ala Pro Val Pro Met Pro
   1 5
<210> 139
   <211> 6
   <212> PRT
   <213> Homo sapiens
<220>
   <221> SOURCE
   <222> 1..6
   <223> /mol_type="protein" /organism="Homo sapiens"
<400> 139
   Ala Ser Leu Ala Lys Gln
   1 5
<210> 140
   <211> 6
   <212> PRT
   <213> Homo sapiens
<220>
   <221> SOURCE
   <222> 1..6
   <223> /mol_type="protein" /organism="Homo sapiens"
<400> 140
   Ala Thr Leu Ala Asp Glu
   1 5
<210> 141
   <211> 6
   <212> PRT
   <213> Homo sapiens
<220>
   <221> SOURCE
   <222> 1..6
   <223> /mol_type="protein" /organism="Homo sapiens"
<400> 141
   Asp Glu Ala Ala Gly His
   1 5
<210> 142
   <211> 6
   <212> PRT
   <213> Homo sapiens
<220>
   <221> SOURCE
   <222> 1..6
   <223> /mol_type="protein" /organism="Homo sapiens"
<400> 142
   Asp Arg Lys Asp Gln Gly
   1 5
<210> 143
   <211> 6
   <212> PRT
   <213> Homo sapiens
<220>
   <221> SOURCE
   <222> 1..6
   <223> /mol_type="protein" /organism="Homo sapiens"
<400> 143
   Glu Ala Ala Gly His Val
   1 5
<210> 144
   <211> 6
   <212> PRT
   <213> Homo sapiens
<220>
   <221> SOURCE
   <222> 1..6
   <223> /mol_type="protein" /organism="Homo sapiens"
<400> 144
   Glu Asp His Ala Gly Thr
   1               5
<210> 145
   <211> 6
   <212> PRT
   <213> Homo sapiens
<220>
   <221> SOURCE
   <222> 1..6
   <223> /mol_type="protein" /organism="Homo sapiens"
<400> 145
   Glu Gly Asp Thr Asp Ala
   1               5
<210> 146
   <211> 6
   <212> PRT
   <213> Homo sapiens
<220>
   <221> SOURCE
   <222> 1..6
   <223> /mol_type="protein" /organism="Homo sapiens"
<400> 146
   Glu Asn Ala Lys Ala Lys
   1               5
<210> 147
   <211> 6
   <212> PRT
   <213> Homo sapiens
<220>
   <221> SOURCE
   <222> 1..6
   <223> /mol_type="protein" /organism="Homo sapiens"
<400> 147
   Glu Val Met Glu Asp His
   1               5
<210> 148
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 3
   <223> oxidised methionine
<400> 148
   Glu Val Met Glu Asp His
   1 5
<210> 149
   <211> 6
   <212> PRT
   <213> Homo sapiens
<220>
   <221> SOURCE
   <222> 1..6
   <223> /mol_type="protein" /organism="Homo sapiens"
<400> 149
   Glu Val Ser Ala Ser Leu
   1 5
<210> 150
   <211> 6
   <212> PRT
   <213> Homo sapiens
<220>
   <221> SOURCE
   <222> 1..6
   <223> /mol_type="protein" /organism="Homo sapiens"
<400> 150
   Gly Ala Ala Pro Pro Gly
   1 5
<210> 151
   <211> 6
   <212> PRT
   <213> Homo sapiens
<220>
   <221> SOURCE
   <222> 1..6
   <223> /mol_type="protein" /organism="Homo sapiens"
<400> 151
   Gly Glu Pro Pro Lys Ser
   1               5
<210> 152
   <211> 6
   <212> PRT
   <213> Homo sapiens
<220>
   <221> SOURCE
   <222> 1..6
   <223> /mol_type="protein" /organism="Homo sapiens"
<400> 152
   Gly Ser Pro Gly Thr Pro
   1               5
<210> 153
   <211> 6
   <212> PRT
   <213> Homo sapiens
<220>
   <221> SOURCE
   <222> 1..6
   <223> /mol_type="protein" /organism="Homo sapiens"
<400> 153
   Gly Thr Pro Gly Ser Arg
   1               5
<210> 154
   <211> 6
   <212> PRT
   <213> Homo sapiens
<220>
   <221> SOURCE
   <222> 1..6
   <223> /mol_type="protein" /organism="Homo sapiens"
<400> 154
   His Gly Ala Glu Ile Val
   1               5
<210> 155
   <211> 6
   <212> PRT
   <213> Homo sapiens
<220>
   <221> SOURCE
   <222> 1..6
   <223> /mol_type="protein" /organism="Homo sapiens"
<400> 155
   His Lys Pro Gly Gly Gly
   1 5
<210> 156
   <211> 6
   <212> PRT
   <213> Homo sapiens
<220>
   <221> SOURCE
   <222> 1..6
   <223> /mol_type="protein" /organism="Homo sapiens"
<400> 156
   Ile Pro Ala Lys Thr Pro
   1 5
<210> 157
   <211> 6
   <212> PRT
   <213> Homo sapiens
<220>
   <221> SOURCE
   <222> 1..6
   <223> /mol_type="protein" /organism="Homo sapiens"
<400> 157
   Lys Ala Lys Thr Asp His
   1 5
<210> 158
   <211> 6
   <212> PRT
   <213> Homo sapiens
<220>
   <221> SOURCE
   <222> 1..6
   <223> /mol_type="protein" /organism="Homo sapiens"
<400> 158
   Lys Ser Lys Asp Gly Thr
<210> 159
   <211> 6
   <212> PRT
   <213> Homo sapiens
<220>
   <221> SOURCE
   <222> 1..6
   <223> /mol_type="protein" /organism="Homo sapiens"
<400> 159
   Lys Ser Pro Val Val Ser
   1 5
<210> 160
   <211> 6
   <212> PRT
   <213> Homo sapiens
<220>
   <221> SOURCE
   <222> 1..6
   <223> /mol_type="protein" /organism="Homo sapiens"
<400> 160
   Lys Thr Pro Pro Ala Pro
   1 5
<210> 161
   <211> 6
   <212> PRT
   <213> Homo sapiens
<220>
   <221> SOURCE
   <222> 1..6
   <223> /mol_type="protein" /organism="Homo sapiens"
<400> 161
   Leu Ala Thr Leu Ala Asp
   1 5
<210> 162
   <211> 6
   <212> PRT
   <213> Homo sapiens
<220>
   <221> SOURCE
   <222> 1..6
   <223> /mol_type="protein" /organism="Homo sapiens"
<400> 162
   Leu Lys Asn Val Lys Ser
   1 5
<210> 163
   <211> 6
   <212> PRT
   <213> Homo sapiens
<220>
   <221> SOURCE
   <222> 1..6
   <223> /mol_type="protein" /organism="Homo sapiens"
<400> 163
   Leu Pro Thr Pro Pro Thr
   1               5
<210> 164
   <211> 6
   <212> PRT
   <213> Homo sapiens
<220>
   <221> SOURCE
   <222> 1..6
   <223> /mol_type="protein" /organism="Homo sapiens"
<400> 164
   Met His Gln Asp Gln Glu
   1               5
<210> 165
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 1
   <223> oxidised methionine
<400> 165
   Met His Gln Asp Gln Glu
   1               5
<210> 166
   <211> 6
   <212> PRT
   <213> Homo sapiens
<220>
   <221> SOURCE
   <222> 1..6
   <223> /mol_type="protein" /organism="Homo sapiens"
<400> 166
   Met Val Asp Ser Pro Gln
   1 5
<210> 167
   <211> 6
   <212> PRT
   <213> Homo sapiens
<220>
   <221> SOURCE
   <222> 1..6
   <223> /mol_type="protein" /organism="Homo sapiens"
<400> 167
   Asn Ala Thr Arg Ile Pro
   1 5
<210> 168
   <211> 6
   <212> PRT
   <213> Homo sapiens
<220>
   <221> SOURCE
   <222> 1..6
   <223> /mol_type="protein" /organism="Homo sapiens"
<400> 168
   Asn Ile His His Lys Pro
   1 5
<210> 169
   <211> 6
   <212> PRT
   <213> Homo sapiens
<220>
   <221> SOURCE
   <222> 1..6
   <223> /mol_type="protein" /organism="Homo sapiens"
<400> 169
   Pro Gly Ser Pro Gly Thr
   1 5
<210> 170
   <211> 6
   <212> PRT
   <213> Homo sapiens
<220>
   <221> SOURCE
   <222> 1..6
   <223> /mol_type="protein" /organism="Homo sapiens"
<400> 170
   Pro Pro Ala Pro Lys Thr
   1 5
<210> 171
   <211> 6
   <212> PRT
   <213> Homo sapiens
<220>
   <221> SOURCE
   <222> 1..6
   <223> /mol_type="protein" /organism="Homo sapiens"
<400> 171
   Pro Pro Lys Ser Gly Asp
   1 5
<210> 172
   <211> 6
   <212> PRT
   <213> Homo sapiens
<220>
   <221> SOURCE
   <222> 1..6
   <223> /mol_type="protein" /organism="Homo sapiens"
<400> 172
   Pro Pro Thr Arg Glu Pro
   1 5
<210> 173
   <211> 6
   <212> PRT
   <213> Homo sapiens
<220>
   <221> SOURCE
   <222> 1..6
   <223> /mol_type="protein" /organism="Homo sapiens"
<400> 173
   Pro Val Pro Met Pro Asp
   1 5
<210> 174
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 4
   <223> oxidised methionine
<400> 174
   Pro Val Pro Met Pro Asp
   1               5
<210> 175
   <211> 6
   <212> PRT
   <213> Homo sapiens
<220>
   <221> SOURCE
   <222> 1..6
   <223> /mol_type="protein" /organism="Homo sapiens"
<400> 175
   Gln Leu Ala Thr Leu Ala
   1               5
<210> 176
   <211> 6
   <212> PRT
   <213> Homo sapiens
<220>
   <221> SOURCE
   <222> 1..6
   <223> /mol_type="protein" /organism="Homo sapiens"
<400> 176
   Gln Thr Ala Pro Val Pro
   1               5
<210> 177
   <211> 6
   <212> PRT
   <213> Homo sapiens
<220>
   <221> SOURCE
   <222> 1..6
   <223> /mol_type="protein" /organism="Homo sapiens"
<400> 177
   Arg Glu Asn Ala Lys Ala
   1 5
<210> 178
   <211> 6
   <212> PRT
   <213> Homo sapiens
<220>
   <221> SOURCE
   <222> 1..6
   <223> /mol_type="protein" /organism="Homo sapiens"
<400> 178
   Arg Ile Pro Ala Lys Thr
   1 5
<210> 179
   <211> 6
   <212> PRT
   <213> Homo sapiens
<220>
   <221> SOURCE
   <222> 1..6
   <223> /mol_type="protein" /organism="Homo sapiens"
<400> 179
   Arg Lys Asp Gln Gly Gly
   1 5
<210> 180
   <211> 6
   <212> PRT
   <213> Homo sapiens
<220>
   <221> SOURCE
   <222> 1..6
   <223> /mol_type="protein" /organism="Homo sapiens"
<400> 180
   Arg Thr Pro Pro Lys Ser
   1 5
<210> 181
   <211> 6
   <212> PRT
   <213> Homo sapiens
<220>
   <221> SOURCE
   <222> 1..6
   <223> /mol_type="protein" /organism="Homo sapiens"
<400> 181
   Arg Thr Pro Ser Leu Pro
   1 5
<210> 182
   <211> 6
   <212> PRT
   <213> Homo sapiens
<220>
   <221> SOURCE
   <222> 1..6
   <223> /mol_type="protein" /organism="Homo sapiens"
<400> 182
   Ser Ala Ser Leu Ala Lys
   1 5
<210> 183
   <211> 6
   <212> PRT
   <213> Homo sapiens
<220>
   <221> SOURCE
   <222> 1..6
   <223> /mol_type="protein" /organism="Homo sapiens"
<400> 183
   Ser Gly Asp Thr Ser Pro
   1 5
<210> 184
   <211> 6
   <212> PRT
   <213> Homo sapiens
<220>
   <221> SOURCE
   <222> 1..6
   <223> /mol_type="protein" /organism="Homo sapiens"
<400> 184
   Ser Gly Glu Pro Pro Lys
   1 5
<210> 185
   <211> 6
   <212> PRT
   <213> Homo sapiens
<220>
   <221> SOURCE
   <222> 1..6
   <223> /mol_type="protein" /organism="Homo sapiens"
<400> 185
   Ser Leu Ala Lys Gln Gly
   1               5
<210> 186
   <211> 6
   <212> PRT
   <213> Homo sapiens
<220>
   <221> SOURCE
   <222> 1..6
   <223> /mol_type="protein" /organism="Homo sapiens"
<400> 186
   Ser Pro Gly Ser Pro Gly
   1               5
<210> 187
   <211> 6
   <212> PRT
   <213> Homo sapiens
<220>
   <221> SOURCE
   <222> 1..6
   <223> /mol_type="protein" /organism="Homo sapiens"
<400> 187
   Ser Pro Gln Leu Ala Thr
   1               5
<210> 188
   <211> 6
   <212> PRT
   <213> Homo sapiens
<220>
   <221> SOURCE
   <222> 1..6
   <223> /mol_type="protein" /organism="Homo sapiens"
<400> 188
   Ser Pro Ser Ser Ala Lys
   1               5
<210> 189
   <211> 6
   <212> PRT
   <213> Homo sapiens
<220>
   <221> SOURCE
   <222> 1..6
   <223> /mol_type="protein" /organism="Homo sapiens"
<400> 189
   Ser Pro Val Val Ser Gly
   1 5
<210> 190
   <211> 6
   <212> PRT
   <213> Homo sapiens
<220>
   <221> SOURCE
   <222> 1..6
   <223> /mol_type="protein" /organism="Homo sapiens"
<400> 190
   Ser Arg Ser Arg Thr Pro
   1 5
<210> 191
   <211> 6
   <212> PRT
   <213> Homo sapiens
<220>
   <221> SOURCE
   <222> 1..6
   <223> /mol_type="protein" /organism="Homo sapiens"
<400> 191
   Ser Arg Thr Pro Ser Leu
   1 5
<210> 192
   <211> 6
   <212> PRT
   <213> Homo sapiens
<220>
   <221> SOURCE
   <222> 1..6
   <223> /mol_type="protein" /organism="Homo sapiens"
<400> 192
   Thr Ala Pro Val Pro Met
   1 5
<210> 193
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 6
   <223> oxidised methionine
<400> 193
   Thr Ala Pro Val Pro Met
   1               5
<210> 194
   <211> 6
   <212> PRT
   <213> Homo sapiens
<220>
   <221> SOURCE
   <222> 1..6
   <223> /mol_type="protein" /organism="Homo sapiens"
<400> 194
   Thr Leu Ala Asp Glu Val
   1               5
<210> 195
   <211> 6
   <212> PRT
   <213> Homo sapiens
<220>
   <221> SOURCE
   <222> 1..6
   <223> /mol_type="protein" /organism="Homo sapiens"
<400> 195
   Thr Pro Pro Lys Ser Pro
   1               5
<210> 196
   <211> 6
   <212> PRT
   <213> Homo sapiens
<220>
   <221> SOURCE
   <222> 1..6
   <223> /mol_type="protein" /organism="Homo sapiens"
<400> 196
   Thr Pro Pro Ser Ser Gly
   1 5
<210> 197
   <211> 6
   <212> PRT
   <213> Homo sapiens
<220>
   <221> SOURCE
   <222> 1..6
   <223> /mol_type="protein" /organism="Homo sapiens"
<400> 197
   Thr Pro Arg Gly Ala Ala
   1 5
<210> 198
   <211> 6
   <212> PRT
   <213> Homo sapiens
<220>
   <221> SOURCE
   <222> 1..6
   <223> /mol_type="protein" /organism="Homo sapiens"
<400> 198
   Thr Pro Ser Leu Glu Asp
   1 5
<210> 199
   <211> 6
   <212> PRT
   <213> Homo sapiens
<220>
   <221> SOURCE
   <222> 1..6
   <223> /mol_type="protein" /organism="Homo sapiens"
<400> 199
   Thr Arg Ile Pro Ala Lys
   1 5
<210> 200
   <211> 6
   <212> PRT
   <213> Homo sapiens
<220>
   <221> SOURCE
   <222> 1..6
   <223> /mol_type="protein" /organism="Homo sapiens"
<400> 200
   Thr Ser Pro Arg His Leu
   1               5
<210> 201
   <211> 6
   <212> PRT
   <213> Homo sapiens
<220>
   <221> SOURCE
   <222> 1..6
   <223> /mol_type="protein" /organism="Homo sapiens"
<400> 201
   Val Pro Gly Gly Gly Asn
   1               5
<210> 202
   <211> 6
   <212> PRT
   <213> Homo sapiens
<220>
   <221> SOURCE
   <222> 1..6
   <223> /mol_type="protein" /organism="Homo sapiens"
<400> 202
   Val Ser Ala Ser Leu Ala
   1               5
<210> 203
   <211> 6
   <212> PRT
   <213> Homo sapiens
<220>
   <221> SOURCE
   <222> 1..6
   <223> /mol_type="protein" /organism="Homo sapiens"
<400> 203
   Ala Ala Gly His Val Thr
   1               5
<210> 204
   <211> 6
   <212> PRT
   <213> Homo sapiens
<220>
   <221> SOURCE
   <222> 1..6
   <223> /mol_type="protein" /organism="Homo sapiens"
<400> 204
   Ala Glu Ile Val Tyr Lys
   1               5
<210> 205
   <211> 6
   <212> PRT
   <213> Homo sapiens
<220>
   <221> SOURCE
   <222> 1..6
   <223> /mol_type="protein" /organism="Homo sapiens"
<400> 205
   Ala Lys Ser Arg Leu Gln
   1               5
<210> 206
   <211> 6
   <212> PRT
   <213> Homo sapiens
<220>
   <221> SOURCE
   <222> 1..6
   <223> /mol_type="protein" /organism="Homo sapiens"
<400> 206
   Ala Pro Pro Gly Gln Lys
   1               5
<210> 207
   <211> 6
   <212> PRT
   <213> Homo sapiens
<220>
   <221> SOURCE
   <222> 1..6
   <223> /mol_type="protein" /organism="Homo sapiens"
<400> 207
   Asp Leu Lys Asn Val Lys
   1 5
<210> 208
   <211> 6
   <212> PRT
   <213> Homo sapiens
<220>
   <221> SOURCE
   <222> 1..6
   <223> /mol_type="protein" /organism="Homo sapiens"
<400> 208
   Asp Gln Gly Gly Tyr Thr
   1 5
<210> 209
   <211> 6
   <212> PRT
   <213> Homo sapiens
<220>
   <221> SOURCE
   <222> 1..6
   <223> /mol_type="protein" /organism="Homo sapiens"
<400> 209
   Asp Arg Ser Gly Tyr Ser
   1 5
<210> 210
   <211> 6
   <212> PRT
   <213> Homo sapiens
<220>
   <221> SOURCE
   <222> 1..6
   <223> /mol_type="protein" /organism="Homo sapiens"
<400> 210
   Glu Pro Lys Lys Val Ala
   1 5
<210> 211
   <211> 6
   <212> PRT
   <213> Homo sapiens
<220>
   <221> SOURCE
   <222> 1..6
   <223> /mol_type="protein" /organism="Homo sapiens"
<400> 211
   Gly Ala Glu Ile Val Tyr
   1 5
<210> 212
   <211> 6
   <212> PRT
   <213> Homo sapiens
<220>
   <221> SOURCE
   <222> 1..6
   <223> /mol_type="protein" /organism="Homo sapiens"
<400> 212
   Gly Gly Gly Gln Val Glu
   1 5
<210> 213
   <211> 6
   <212> PRT
   <213> Homo sapiens
<220>
   <221> SOURCE
   <222> 1..6
   <223> /mol_type="protein" /organism="Homo sapiens"
<400> 213
   Gly Gly Gly Ser Val Gln
   1 5
<210> 214
   <211> 6
   <212> PRT
   <213> Homo sapiens
<220>
   <221> SOURCE
   <222> 1..6
   <223> /mol_type="protein" /organism="Homo sapiens"
<400> 214
   Gly Lys Thr Lys Ile Ala
   1 5
<210> 215
   <211> 6
   <212> PRT
   <213> Homo sapiens
<220>
   <221> SOURCE
   <222> 1..6
   <223> /mol_type="protein" /organism="Homo sapiens"
<400> 215
   Gly Asn Lys Lys Ile Glu
   1 5
<210> 216
   <211> 6
   <212> PRT
   <213> Homo sapiens
<220>
   <221> SOURCE
   <222> 1..6
   <223> /mol_type="protein" /organism="Homo sapiens"
<400> 216
   Gly Gln Ala Asn Ala Thr
   1 5
<210> 217
   <211> 6
   <212> PRT
   <213> Homo sapiens
<220>
   <221> SOURCE
   <222> 1..6
   <223> /mol_type="protein" /organism="Homo sapiens"
<400> 217
   Gly Gln Val Glu Val Lys
   1 5
<210> 218
   <211> 6
   <212> PRT
   <213> Homo sapiens
<220>
   <221> SOURCE
   <222> 1..6
   <223> /mol_type="protein" /organism="Homo sapiens"
<400> 218
   Gly Ser Arg Ser Arg Thr
   1 5
<210> 219
   <211> 6
   <212> PRT
   <213> Homo sapiens
<220>
   <221> SOURCE
   <222> 1..6
   <223> /mol_type="protein" /organism="Homo sapiens"
<400> 219
   His Ala Gly Thr Tyr Gly
   1 5
<210> 220
   <211> 6
   <212> PRT
   <213> Homo sapiens
<220>
   <221> SOURCE
   <222> 1..6
   <223> /mol_type="protein" /organism="Homo sapiens"
<400> 220
   Ile Asn Lys Lys Leu Asp
   1 5
<210> 221
   <211> 6
   <212> PRT
   <213> Homo sapiens
<220>
   <221> SOURCE
   <222> 1..6
   <223> /mol_type="protein" /organism="Homo sapiens"
<400> 221
   Lys Ala Lys Gly Ala Asp
   1 5
<210> 222
   <211> 6
   <212> PRT
   <213> Homo sapiens
<220>
   <221> SOURCE
   <222> 1..6
   <223> /mol_type="protein" /organism="Homo sapiens"
<400> 222
   Lys Lys Val Ala Val Val
   1 5
<210> 223
   <211> 6
   <212> PRT
   <213> Homo sapiens
<220>
   <221> SOURCE
   <222> 1..6
   <223> /mol_type="protein" /organism="Homo sapiens"
<400> 223
   Lys Ser Pro Ser Ser Ala
   1 5
<210> 224
   <211> 6
   <212> PRT
   <213> Homo sapiens
<220>
   <221> SOURCE
   <222> 1..6
   <223> /mol_type="protein" /organism="Homo sapiens"
<400> 224
   Leu Gly Asp Arg Lys Asp
   1 5
<210> 225
   <211> 6
   <212> PRT
   <213> Homo sapiens
<220>
   <221> SOURCE
   <222> 1..6
   <223> /mol_type="protein" /organism="Homo sapiens"
<400> 225
   Met Pro Asp Leu Lys Asn
   1 5
<210> 226
   <211> 6
   <212> PRT
   <213> Homo sapiens
<220>
   <221> SOURCE
   <222> 1..6
   <223> /mol_type="protein" /organism="Homo sapiens"
<400> 226
   Asn Lys Lys Ile Glu Thr
   1               5
<210> 227
   <211> 6
   <212> PRT
   <213> Homo sapiens
<220>
   <221> SOURCE
   <222> 1..6
   <223> /mol_type="protein" /organism="Homo sapiens"
<400> 227
   Pro Gly Ser Arg Ser Arg
   1               5
<210> 228
   <211> 6
   <212> PRT
   <213> Homo sapiens
<220>
   <221> SOURCE
   <222> 1..6
   <223> /mol_type="protein" /organism="Homo sapiens"
<400> 228
   Pro Lys Ser Gly Asp Arg
   1               5
<210> 229
   <211> 6
   <212> PRT
   <213> Homo sapiens
<220>
   <221> SOURCE
   <222> 1..6
   <223> /mol_type="protein" /organism="Homo sapiens"
<400> 229
   Pro Lys Thr Pro Pro Ser
   1               5
<210> 230
   <211> 6
   <212> PRT
   <213> Homo sapiens
<220>
   <221> SOURCE
   <222> 1..6
   <223> /mol_type="protein" /organism="Homo sapiens"
<220>
   <221> MOD_RES
   <222> 2
   <223> oxidised methionine
<400> 230
   Pro Met Pro Asp Leu Lys
   1               5
<210> 231
   <211> 6
   <212> PRT
   <213> Homo sapiens
<220>
   <221> SOURCE
   <222> 1..6
   <223> /mol_type="protein" /organism="Homo sapiens"
<400> 231
   Pro Thr Pro Pro Thr Arg
   1               5
<210> 232
   <211> 6
   <212> PRT
   <213> Homo sapiens
<220>
   <221> SOURCE
   <222> 1..6
   <223> /mol_type="protein" /organism="Homo sapiens"
<400> 232
   Pro Thr Arg Glu Pro Lys
   1               5
<210> 233
   <211> 6
   <212> PRT
   <213> Homo sapiens
<220>
   <221> SOURCE
   <222> 1..6
   <223> /mol_type="protein" /organism="Homo sapiens"
<400> 233
   Gln Asp Gln Glu Gly Asp
   1               5
<210> 234
   <211> 6
   <212> PRT
   <213> Homo sapiens
<220>
   <221> SOURCE
   <222> 1..6
   <223> /mol_type="protein" /organism="Homo sapiens"
<400> 234
   Gln Glu Gly Asp Thr Asp
   1 5
<210> 235
   <211> 6
   <212> PRT
   <213> Homo sapiens
<220>
   <221> SOURCE
   <222> 1..6
   <223> /mol_type="protein" /organism="Homo sapiens"
<400> 235
   Gln Lys Gly Gln Ala Asn
   1 5
<210> 236
   <211> 6
   <212> PRT
   <213> Homo sapiens
<220>
   <221> SOURCE
   <222> 1..6
   <223> /mol_type="protein" /organism="Homo sapiens"
<400> 236
   Arg Ser Arg Thr Pro Ser
   1 5
<210> 237
   <211> 6
   <212> PRT
   <213> Homo sapiens
<220>
   <221> SOURCE
   <222> 1..6
   <223> /mol_type="protein" /organism="Homo sapiens"
<400> 237
   Ser Ala Lys Ser Arg Leu
   1 5
<210> 238
   <211> 6
   <212> PRT
   <213> Homo sapiens
<220>
   <221> SOURCE
   <222> 1..6
   <223> /mol_type="protein" /organism="Homo sapiens"
<400> 238
   Ser Asn Val Gln Ser Lys
   1 5
<210> 239
   <211> 6
   <212> PRT
   <213> Homo sapiens
<220>
   <221> SOURCE
   <222> 1..6
   <223> /mol_type="protein" /organism="Homo sapiens"
<400> 239
   Ser Thr Gly Ser Ile Asp
   1 5
<210> 240
   <211> 6
   <212> PRT
   <213> Homo sapiens
<220>
   <221> SOURCE
   <222> 1..6
   <223> /mol_type="protein" /organism="Homo sapiens"
<400> 240
   Thr Asp Ala Gly Leu Lys
   1 5
<210> 241
   <211> 6
   <212> PRT
   <213> Homo sapiens
<220>
   <221> SOURCE
   <222> 1..6
   <223> /mol_type="protein" /organism="Homo sapiens"
<400> 241
   Thr Pro Gly Ser Arg Ser
   1 5
<210> 242
   <211> 6
   <212> PRT
   <213> Homo sapiens
<220>
   <221> SOURCE
   <222> 1..6
   <223> /mol_type="protein" /organism="Homo sapiens"
<400> 242
   Val Asp Ser Pro Gln Leu
   1 5
<210> 243
   <211> 6
   <212> PRT
   <213> Homo sapiens
<220>
   <221> SOURCE
   <222> 1..6
   <223> /mol_type="protein" /organism="Homo sapiens"
<400> 243
   Val Pro Met Pro Asp Leu
   1 5
<210> 244
   <211> 6
   <212> PRT
   <213> Homo sapiens
<220>
   <221> SOURCE
   <222> 1..6
   <223> /mol_type="protein" /organism="Homo sapiens"
<400> 244
   Val Ser Lys Ser Lys Asp
   1 5
<210> 245
   <211> 6
   <212> PRT
   <213> Homo sapiens
<220>
   <221> SOURCE
   <222> 1..6
   <223> /mol_type="protein" /organism="Homo sapiens"
<400> 245
   Tyr Thr Met His Gln Asp
   1               5
<210> 246
   <211> 10
   <212> PRT
   <213> Homo sapiens
<220>
   <221> SOURCE
   <222> 1..10
   <223> /mol_type="protein" /organism="Homo sapiens"
<400> 246
   Thr Pro Arg Gly Ala Ala Pro Pro Gly Gln
   1               5                   10
<210> 247
   <211> 11
   <212> PRT
   <213> Homo sapiens
<220>
   <221> SOURCE
   <222> 1..11
   <223> /mol_type="protein" /organism="Homo sapiens"
<400> 247
   Ala Thr Pro Arg Gly Ala Ala Pro Pro Gly Gln
   1               5                   10
<210> 248
   <211> 13
   <212> PRT
   <213> Homo sapiens
<220>
   <221> SOURCE
   <222> 1..13
   <223> /mol_type="protein" /organism="Homo sapiens"
<400> 248
   Thr Pro Arg Gly Ala Ala Pro Pro Gly Gln Gly Gly Cys
   1               5                   10

## Description

The present invention relates to assays for biomarkers useful for assessment of cardiovascular events, including but not limited to Acute Myocardial Infarction (AMI), high coronary calcification, and symptomatic non AMI. In particular, according to the present invention, biomarkers relating to degradation fragments of Titin are found to be useful.

Titin, also known as connectin, is a sarcomeric protein expressed in cardiac and skeletal muscle. It is the largest known mammal protein, having a size that can reach up to 3700kDa¹. Its main function and one that is well described is to act as a long molecular spring by restoring passive tension during myocardial stretch²⁻⁴. Titin has two isoforms that are co-expressed in the sarcomere, the N2A which is the larger of the two and is found in both skeletal and myocardial muscle, and the N2B isoform which is smaller, stiffer and is solely found in cardiac muscle ^{1,5-7}. Due to the different stiffness properties of the isoforms of Titin it has been proposed that the adaptive or maladaptive ratio alteration between the two isoforms, in synergy with their corresponding kinase region, during pathologic events could be responsible for affecting the myocardial contractile properties^{3,5, 8-12}. Isoform modifications and ratio alterations have been first described in animal models while clinical studies have also reported isoform shifts during dilated cardiomyopathy (DCM), aortic stenosis (AS), diastolic heart failure (DHF) and ischemic heart disease (IHD)^{11, 13-16}. The main limitation of Titin relevant studies lies on the available methods for detecting and quantifying Titin isoform levels, which are either based on methods that are not sensitive enough such as immunoblotting and gel electrophoresis or techniques which can provide higher sensitivity such as quantitative RT-PCR but can only provide information about the total amount of Titin isoform levels. All of these methods rely on invasive means of tissue collection and identification. Low sensitivity and specificity might contribute to the poor utilisation of these methods as diagnostic or prognostic biomarkers and restrain the correlation of Titin isoform levels with functional studies. In contrast, we discovered according to the present invention that detection of specific degradation fragments of Titin has excellent clinical utility in a number of important clinical situations.

Extracellular matrix (ECM) components are degraded by a number of different proteases including matrix metalloproteinases (MMPs). MMP derived degradation of proteins generates specific cleavage sites/fragments which in turn produce new epitopes. We have previously described that neo epitopes may have potential utility as biomarkers of unbalanced ECM remodeling in a number of different pathologies and can be measured in an array of biological fluids such as serum, plasma and urine ¹⁷⁻²². A key benefit of this approach is that it is a non-invasive method of measuring specific neo epitopes that represent a unique 'fingerprint' of the proteolytic cleavage of the protein and directly reflect specific tissue turnover in both physiology and pathology.

Karsdal et at (Clin. Biochem., 2010, 43; 793-804) reviews the potential usefulness of neo-epitope biomarkers, and discusses how the identification of each modification (i.e. neo-epitope) and the corresponding affected tissue-specific protein may produce unique disease-specific biochemical markers.

Accordingly, the present invention now provides in a first aspect a method of bioassay for the quantification of peptide fragments comprising a neo-epitope formed in by cleavage of a titin protein at a cleavage site by a proteinase, said method comprising contacting a sample comprising said peptide fragments with an immunological binding partner having specific binding affinity for a said neo-epitope and determining the level of binding of said immunological binding partner to peptide fragments in said sample, wherein said immunological binding partner has specific binding affinity for the following sequence at the C terminal of a peptide: **NVTVEARLIK↓ '13542 SEQ ID NO:** 2, wherein the proteinase cleavage site is marked ↓.

The titin fragment detected may be in samples derived from mice and humans. Generally, the fragment detected will be formed in nature and will be naturally occurring in said sample.

Said immunological binding partner has a specific binding affinity for peptide fragments comprising a C-terminal neo-epitope of said titin protein formed by cleavage of a titin protein by a protease.

Said immunological binding partner has specific binding affinity for the following sequence at the C terminal of a peptide: NVTVEARLIK↓ '13542.

Preferably, said immunological binding partner is a monoclonal antibody or a fragment of a monoclonal antibody having specific binding affinity.

Said method is preferably conducted as a competition immunoassay in which said immunological binding partner and a competition agent are incubated in the presence of said sample and the competition agent competes with the peptide fragments in the sample to bind to the immunological binding partner. Optionally, said competition agent is a synthetic peptide or is a purified native peptide formed by cleavage of the protein from which said epitope comes so as to reveal said neo-epitope.

The sample is preferably a sample of urine, serum, blood, plasma, or saliva. Included in the invention are methods in which the sample is a patient derived sample, said method further comprising comparing the determined level of said binding of said peptide fragments with values characteristic of (a) comparable healthy individuals and/or (b) a pathological cardiac condition.

In a further aspect, the invention includes an immunological binding partner against a C-terminal neo-epitope formed by proteinase cleavage of a titin protein.

The immunological binding partner is specifically immunoreactive with the C-terminal of the amino acid sequence:
NVTEARLIK↓ '13542

The immunological binding partner may be a monoclonal antibody or a binding fragment thereof.

The invention includes in a further aspect a cell line producing a monoclonal antibody described above.

In a further aspect, the invention provides a peptide comprising a C-terminal neo-epitope formed by cleavage of a titin protein by a protease in the partial sequence of said proteins set out above. The peptide may be conjugated as a hapten to a carrier for producing an immune response to said peptide, or immobilised to a solid surface or conjugated to a detectable marker for use in an immunoassay.

In a further aspect, the invention provides an isolated nucleic acid molecule coding for a peptide comprising a C-terminal neo-epitope formed by cleavage of a said protein by a protease in the partial sequence of said proteins set out above.

In a still further aspect, the invention provides a vector comprising a nucleic acid sequence comprising an expression signal and a coding sequence which codes for the expression of a peptide comprising a C-terminal neo-epitope formed by cleavage of a said protein by a protease in the partial sequence of said proteins set out above.

In a still further aspect, the invention provides a host cell transformed with a vector as described above and expressing a said peptide.

In a still further aspect, the invention provides an immunoassay kit comprising an immunological binding partner as described above, and a competition agent which binds said immunological binding partner, and optionally one or more of a wash reagent, a buffer, a stopping reagent, an enzyme label, an enzyme label substrate, calibration standards, an anti-mouse antibody and instructions for conducting an assay using said kit.

During digestion of human tissue with an array of exogenous metalloproteases, a large number of proteolyzed peptide products were identified using mass spectrometry²³. Among these, a Titin specific fragment 12670' NVTVEARLIK 12679' was identified to be MMP12 cleavage specific. Proteomic analysis revealed that the sequence is unique for the N2B Titin isoform and homologous to human and mouse. Even though at least in murine models MMP12 has been implicated in cardiovascular events such as atherogenesis, the only MMP previously described to have an effect on Titin degradation in relevant bibliography is MMP2 located in the Z-disk region of cardiac sarcomere, contributing to Titin degradation in ischemic and reperfusion related events^{24, 25}. Due to our mass spectrometry derived finding, we hypothesized that MMP12 may be a more active participant in related pathologic events than previously described and we set out with the hypothesis that the MMP12 specific fragment of the N2B isoform of Titin identified with mass spectrometry could be potentially useful for monitoring pathologic cardiovascular events.

According to the present invention, the following peptide sequence and cleavage site is useful;
Protease Cleavage sites marked ↓
MMP12 13532' NVTVEARLIK↓ '13542

The present invention will be further described and illustrated by the following examples in which reference is made to the accompanying drawings, in which:
Figure 1 shows the reactivity of a monoclonal antibody of the invention to target peptide as measured in Example 1 below;
Figure 2 shows the reactivity of the monoclonal antibody to human serum and urine samples as determined in Example 2; and
Figure 3 shows measurements of titin fragment content in human serum from control patients and patients suffering from cardiac diseases as obtained in Example 3.

### Example 1

### Development of monoclonal antibodies to Titin fragments

All reagents used for experiments were standard high-quality chemicals from Merck (Whitehouse Station, NJ, USA) and Sigma Aldrich (St.Louis, MO, USA). The synthetic peptides used for monoclonal antibody production were purchased from the Chinese Peptide Company, Beijing, China.

### Selection of peptide for immunization

The sequence of the peptide selected for the assay was chosen on the basis of mass spectrometry performed on human tissue²³. Peptide fragments were identified using Uniprot (accession number C0JYZ2). The first 10 amino acids of each free end of the sequences identified were regarded as a target sequences. All relevant sequences were analyzed for homology and then blasted for homology using the CHECK XX NPS@: network protein sequence analysis²⁸. The sequence NVTVEARLIK located between amino acid position 12670' and 12679' (Titin) was selected as immunogen. The sequence was identified by Uniprot and PBIL network protein sequence analysis as being unique to human and mouse Titin. The selected sequence was also found to be present in 6 out of 8 Titin isoforms produced by alternative splicing. These were, isoforms 3 (small cardiac N2-B), 7 (cardiac novex-2) and 8 (cardiac novex-1) that are known to present in cardiac muscle (Uniprot accession numbers Q8WZ42-3, Q8WZ42-7 and Q8WZ42-8) and isoforms 2, 4 and 5 (Uniprot accession numbers Q8WZ42-2, Q8WZ42-4 and Q8WZ42-5)^{26, 27}.

### Immunization procedure

Six 4-6 week old Balb/C mice were immunized subcutaneously in the abdomen with 200 µL emulsified antigen (50 pg per immunization), using Freund's incomplete adjuvant (KLH-CGG-NVTVEARLIK SEQ ID NO: 7). Immunizations were performed at two-week intervals until stable titer levels were obtained. At each bleeding, the serum antibody titer was measured and the mice with the highest antibody titer and best reactivity towards serum and urine were selected for fusion. The selected mice were boosted intravenously with 50 pg immunogen in 100 µL 0.9% sodium chloride solution three days before surgical removal of the spleen for cell fusion.

### Fusion and antibody screening

The fusion procedure has been described elsewhere²⁹. Briefly, mouse spleen cells were fused with SP2/0 myeloma fusion partner cells. The hybridoma cells were cloned using a limiting dilution method and transferred into 96-well microtiter plates for further growth. Standard limited dilution was used to promote monoclonal growth. Supernatants were screened using an indirect ELISA, while the biotinylated peptide Biotin-CGG-NVTVEARLIK was used as a catcher peptide on streptavidin-coated microtitre plates.

### Characterization of clones

Native reactivity and peptide binding of the monoclonal antibodies in human serum, plasma and urine was evaluated using a preliminary ELISA with a 10 ng/mL biotinylated peptide coater on a streptavidin-coated microtitre plate and the supernatant from the growing monoclonal hybridoma. Clone specificity was tested against a free peptide (NVTVEARLIK) and a non-sense peptide. Isotyping of the monoclonal antibodies was performed using the Clonotyping System-HRP kit, cat.5300-05 (Southern Biotech, Birmingham, AL, USA). The selected clones were purified using Protein G columns according to the manufacturer's instructions and dialysed (GE Healthcare Life Science, Little Chalfont, Buckinghamshire, UK).

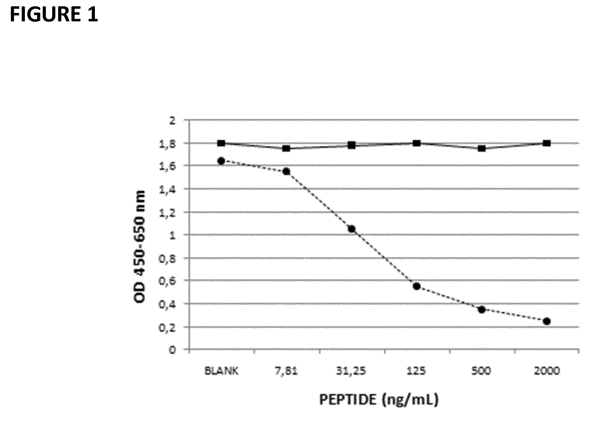

In Figure 1 the reactivity of a monoclonal antibody against the target sequence NVTVEARLIK (circles, dashed line) is compared to the de-selection (and C-terminally elongated) sequence NVTVEARLIKV (square, solid line). The data clearly demonstrate a strong reactivity to the target sequence NVTVEARLIK with no detectable binding to the elongated peptide. These data suggest a strong binding affinity with the sequence having a free C-terminal lysine residue, i.e. the neo-epitope specificity of the monoclonal antibody is confirmed.

### Example 2

### MMP12 Titin assay protocol

The following competitive ELISA protocol was optimised for use with the MMP12 Titin monoclonal antibody. The selected monoclonal antibodies were labelled with horseradish peroxidase (HRP) using the Lightning-Link Horseradish Peroxidase (HRP) antibody labelling kit according to the manufacturer's instructions (Innovabioscience, Babraham, Cambridge, UK). A 96-well streptavidin plate (Roche diagnostics, Basel, Switzerland) was coated with 6.8ng of the biotinylated synthetic peptide, Biotin-CGG-NVTVEARLIK, dissolved in assay buffer 25mM Tris BTB and incubated for 30 minutes at 4°C. 20µL of the peptide calibrator or sample were added to appropriate wells, followed by 100 µL of 280ng conjugated monoclonal antibody and incubated for 1 hour at 4°C. Finally, 100 µL tetramethyl benzinidine (TMB) (Kem-En-Tec cat.4380H, Taastrup, Denmark) was added, and the plate was incubated for 15 minutes at 20°C in the dark. All the above incubation steps included shaking at 300 rpm. After each incubation step the plate was washed five times in washing buffer (20 mM Tris, 50 mM NaCl, pH 7.2). The TMB reaction was stopped by adding 100 µL of stopping solution (1% HCl) and measured at 450 nm with 650 nm as the reference. A calibration curve was plotted using a 4-parametric mathematical fit model with a starting concentration of 200 ng for the standard peptide following a 2-fold dilution.

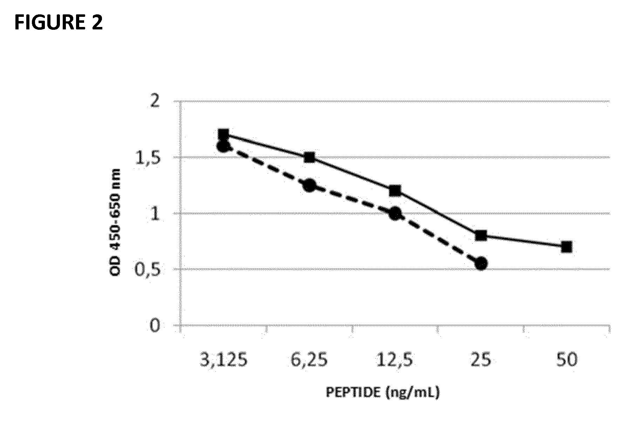

In Figure 2 the reactivity to titin fragments present in human serum is demonstrated. A human serum sample obtained from an individual with AMI was diluted 2-fold and incubated in the MMP12 Titin Elisa described above. The similarity of the curve obtained with human serum and the standard curve suggest that the affinity of the monoclonal antibody is similar against the synthetic peptide NVTVEARLIK and the fragments present in human serum.

### Example 3

### Assessment of serum samples from subjects with cardiovascular events in the MMP12 Titin ELISA

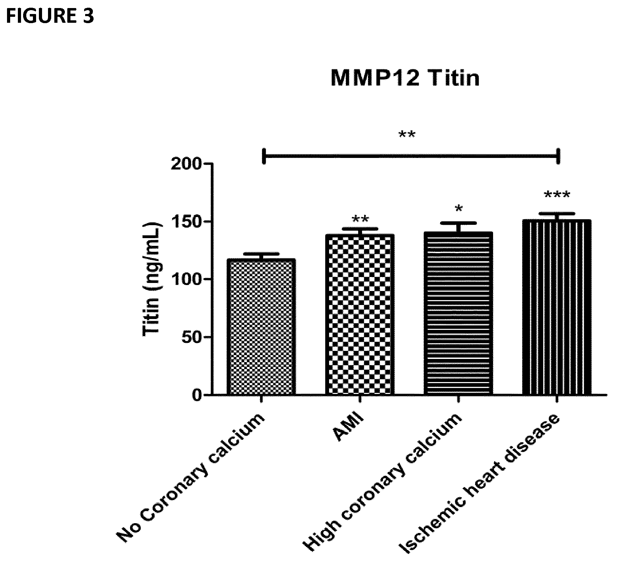

Serum from subjects respectively diagnosed with Acute Myocardial Infarction (AMI), high coronary calcification, and symptomatic non AMI were tested in the MMP12 Titin ELISA and compared to healthy controls (Figure 3).

A statistically significant increase of the marker was measured for all three patient groups. Mean level of controls was measured to be 116.6ng/ml. MMP12 Titin levels were elevated in all patient groups examined, AMI patients mean value 137.9ng/ml (P<0.05), Patients with high coronary calcification 140ng/ml (P<0.05) and Ischemic heart disease 150.6ng/ml (P<0.05) (Figure 3).

Biochemical markers consisting of protein fragments from ECM degradation may be informative of disease pathology and progression, which in turn may be useful for diagnostic and prognostic purposes. These markers could potentially detect changes resulting from intervention strategies and serve as surrogate markers of drug efficacy³⁰.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'. No acknowledgement of any prior published document herein should be taken to be an admission or representation that the teaching thereof was common general knowledge in Australia or elsewhere at the date hereof.

### Reference List

(1) LeWinter MM, Wu Y, Labeit S, Granzier H. Cardiac titin: structure, functions and role in disease. Clin Chim Acta 2007 January;375(1-2):1-9.
(2) LeWinter MM, Granzier H. Cardiac titin: a multifunctional giant. Circulation 2010 May 18;121(19):2137-45.
(3) Wu Y, Bell SP, Trombitas K et al. Changes in titin isoform expression in pacing-induced cardiac failure give rise to increased passive muscle stiffness. Circulation 2002 September 10;106(11):1384-9.
(4) Sutko JL, Publicover NG, Moss RL. Titin: an elastic link between length and active force production in myocardium. Circulation 2001 October 2;104(14):1585-7.
(5) Jaber WA, Maniu C, Krysiak J et al. Titin isoforms, extracellular matrix, and global chamber remodeling in experimental dilated cardiomyopathy: functional implications and mechanistic insight. Circ Heart Fail 2008 September;1(3):192-9.
(6) Hein S, Gaasch WH, Schaper J. Giant molecule titin and myocardial stiffness. Circulation 2002 September 10;106(11):1302-4.
(7) LeWinter MM. Titin isoforms in heart failure: are there benefits to supersizing? Circulation 2004 July 13;110(2):109-11.
(8) Linke WA. Sense and stretchability: the role of titin and titin-associated proteins in myocardial stress-sensing and mechanical dysfunction. Cardiovasc Res 2008 March 1;77(4):637-48.
(9) Chaturvedi RR, Herron T, Simmons R et al. Passive stiffness of myocardium from congenital heart disease and implications for diastole. Circulation 2010 March 2;121(8):979-88.
(10) Thijssen VL, Borgers M, Lenders MH et al. Temporal and spatial variations in structural protein expression during the progression from stunned to hibernating myocardium. Circulation 2004 November 23;110(21):3313-21.
(11) Nagueh SF, Shah G, Wu Y et al. Altered titin expression, myocardial stiffness, and left ventricular function in patients with dilated cardiomyopathy. Circulation 2004 July 13;110(2):155-62.
(12) Peng J, Raddatz K, Molkentin JD et al. Cardiac hypertrophy and reduced contractility in hearts deficient in the titin kinase region. Circulation 2007 February 13;115(6):743-51.
(13) Linke WA. Titin stiffness in heart disease. Circulation 2003 March 25;107(11):e73.
(14) Neagoe C, Kulke M, del MF et al. Titin isoform switch in ischemic human heart disease. Circulation 2002 September 10;106(11):1333-41.
(15) Makarenko I, Opitz CA, Leake MC et al. Passive stiffness changes caused by upregulation of compliant titin isoforms in human dilated cardiomyopathy hearts. Circ Res 2004 October 1;95(7):708-16.
(16) van HL, Borbely A, Niessen HW et al. Myocardial structure and function differ in systolic and diastolic heart failure. Circulation 2006 April 25;113(16):1966-73.
(17) Karsdal MA, Henriksen K, Leeming DJ et al. Biochemical markers and the FDA Critical Path: how biomarkers may contribute to the understanding of pathophysiology and provide unique and necessary tools for drug development. Biomarkers 2009 May;14(3):181-202.
(18) Vassiliadis E, Veidal SS, Simonsen H et al. Immunological detection of the type V collagen propeptide fragment, PVCP-1230, in connective tissue remodeling associated with liver fibrosis 1. Biomarkers 2011 May 25.
(19) Vassiliadis E, Larsen DV, Clausen RE et al. Measurement of C03-610, a Potential Liver Biomarker Derived from Matrix Metalloproteinase-9 Degradation of Collagen Type III, in a Rat Model of Reversible Carbon-Tetrachloride-Induced Fibrosis 4. Biomark Insights 2011;6:49-58.
(20) Vassiliadis E, Veidal SS, Barascuk N et al. Measurement of matrix metalloproteinase 9-mediated collagen type III degradation fragment as a marker of skin fibrosis 5. BMC Dermatol 2011;11:6.
(21) Veidal SS, Vassiliadis E, Bay-Jensen AC, Tougas G, Vainer B, Karsdal MA. Procollagen type I N-terminal propeptide (PINP) is a marker for fibrogenesis in bile duct ligation-induced fibrosis in rats. Fibrogenesis Tissue Repair 2010;3(1):5.
(22) Barascuk N, Vassiliadis E, Larsen L et al. Development and validation of an enzyme-linked immunosorbent assay for the quantification of a specific MMP-9 mediated degradation fragment of type III collagen-A novel biomarker of atherosclerotic plaque remodeling. Clin Biochem 2011 July;44(10-11):900-6.
(23) Zhen EY, Brittain IJ, Laska DA et al. Characterization of metalloprotease cleavage products of human articular cartilage. Arthritis Rheum 2008 August;58(8):2420-31.
(24) Ali MA, Cho WJ, Hudson B, Kassiri Z, Granzier H, Schulz R. Titin is a target of matrix metalloproteinase-2: implications in myocardial ischemia/reperfusion injury. Circulation 2010 November 16;122(20):2039-47.
(25) Johnson JL, George SJ, Newby AC, Jackson CL. Divergent effects of matrix metalloproteinases 3, 7, 9, and 12 on atherosclerotic plaque stability in mouse brachiocephalic arteries. Proc Natl Acad Sci U S A 2005 October 25;102(43):15575-80.
(26) Bang M-L, Centner R, Fornoff F et al. The complete gene sequence of titin, expression of an unusual ∼700 kDa titin isoform and its interaction with obscurin identify a novel Z-line to I-band linking system. Circ Res 2001;89:1065-72.
(27) Musco G, Tziatzos C, Schuck P, Pastore A. Dissecting titin into its structural motifs: identification of an alpha helix near the N-terminus. Biochemistry 1995;34:553-61.
(28) Combet C, Blanchet C, Geourjon C, Deleage G. NPS@: network protein sequence analysis. Trends Biochem Sci 2000 March;25(3):147-50.
(29) Gefter ML, Margulies DH, Scharff MD. A simple method for polyethylene glycol-promoted hybridization of mouse myeloma cells. Somatic Cell Genet 1977 March;3(2):231-6.
(30) Munoz-Luque J, Ros J, Fernandez-Varo G et al. Regression of fibrosis after chronic stimulation of cannabinoid CB2 receptor in cirrhotic rats. J Pharmacol Exp Ther 2008 February;324(2):475-83.

## Claims

1. A method of bioassay for the quantification of peptide fragments comprising a neo-epitope formed by cleavage of a titin protein at a cleavage site by a proteinase, said method comprising contacting a sample comprising said peptide fragments with an immunological binding partner having specific binding affinity for said neo-epitope and determining the level of binding of said immunological binding partner to peptide fragments in said sample, wherein said immunological binding partner has specific binding affinity for the following sequence at the C terminal of a peptide: **NVTVEARLIK↓ '13542 SEQ ID NO: 2,** wherein the proteinase cleavage site is marked ↓.

2. A method as claimed in any preceding claim, wherein said method is conducted as a competition immunoassay in which said immunological binding partner and a competition agent are incubated in the presence of said sample and the competition agent competes with the peptide fragments in the sample to bind to the immunological binding partner.

3. A method as claimed in any preceding claim, wherein the sample is a sample of urine, serum, blood, plasma, or saliva.

4. A method as claimed in any preceding claim, wherein the sample is a patient derived sample, said method further comprising comparing the determined level of said binding of said peptide fragments with values characteristic of (a) comparable healthy individuals and/or (b) a pathological cardiac condition.

5. An immunological binding partner against a C-terminal neo-epitope formed by proteinase cleavage of a titin protein, which is specifically immunoreactive with the C-terminal of the amino acid sequence: **NVTVEARLIK↓ '13542 SEQ ID NO: 2,** wherein the proteinase cleavage site is marked ↓.

6. A cell line producing a monoclonal antibody being an immunological binding partner as claimed in claim 5.

7. A peptide comprising a C-terminal neo-epitope formed by cleavage of a titin protein by a protease in the partial sequence of said protein set out in claim 1.

8. An isolated nucleic acid molecule coding for a peptide comprising a C-terminal neo-epitope formed by cleavage of a titin protein by a protease in the partial sequence of said protein set out in claim 1.

9. A vector comprising a nucleic acid sequence comprising an expression signal and a coding sequence which codes for the expression of a peptide comprising a C-terminal neo-epitope formed by cleavage of a titin protein by a protease in the partial sequence of said protein set out in claim 1 or a host cell transformed with such a vector and expressing a said peptide.

10. An immunoassay kit comprising an immunological binding partner as claimed in claim 5, and
a competition agent which binds said immunological binding partner, and optionally one or more of a wash reagent, a buffer, a stopping reagent, an enzyme label, an enzyme label substrate, calibration standards, an anti-mouse antibody and instructions for conducting an assay using said kit.

## Patentansprüche

1. Biotestverfahren zur Quantifizierung von Peptidfragmenten, umfassend ein Neoepitop, das durch Spaltung eines Titinproteins an einer Spaltstelle durch eine Proteinase gebildet wird, wobei man bei dem Verfahren eine die Peptidfragmente umfassende Probe mit einem immunologischen Bindungspartner mit spezifischer Bindungsaffinität zu dem Neoepitop in Kontakt bringt und den Grad der Bindung des immunologischen Bindungspartners an Peptidfragmente in der Probe bestimmt, wobei der immunologische Bindungspartner spezifische Bindungsaffinität zu der folgenden Sequenz am C-Terminus eines Peptids aufweist: NVTVEARLIK↓ '13542 SEQ ID NO: 2, wobei die Proteinase-Spaltstelle mit ↓ gekennzeichnet ist.

2. Verfahren nach einem vorhergehenden Anspruch, wobei das Verfahren als kompetitiver Immuntest durchgeführt wird, bei dem der immunologische Bindungspartner und ein konkurrierendes Mittel in Gegenwart der Probe inkubiert werden und das konkurrierende Mittel mit den Peptidfragmenten in der Probe um die Bindung an den immunologischen Bindungspartner konkurriert.

3. Verfahren nach einem vorhergehenden Anspruch, wobei es sich bei der Probe um eine Urin-, Serum-, Blut-, Plasma- oder Speichelprobe handelt.

4. Verfahren nach einem vorhergehenden Anspruch, wobei die Probe eine von einem Patienten stammende Probe ist, wobei man bei dem Verfahren ferner den bestimmten Grad der Bindung der Peptidfragmente mit Werten vergleicht, die für (a) vergleichbare gesunde Individuen und/oder (b) ein pathologisches Herzleiden charakteristisch sind.

5. Immunologischer Bindungspartner gegen ein C-terminales Neoepitop, das durch Proteinase-Spaltung eines Titinproteins gebildet wird, das spezifisch immunreaktiv ist mit dem C-Terminus der Aminosäuresequenz: NVTVEARLIK↓ '13542 SEQ ID NO: 2, wobei die Proteinase-Spaltstelle mit ↓ gekennzeichnet ist.

6. Zelllinie, die einen monoklonalen Antikörper produziert, bei dem es sich um einen immunologischen Bindungspartner nach Anspruch 5 handelt.

7. Peptid, umfassend ein C-terminales Neoepitop, das durch Spaltung eines Titinproteins durch eine Protease in die Teilsequenz des Proteins gemäß Anspruch 1 gebildet wird.

8. Isoliertes Nukleinsäuremolekül, das für ein Peptid, umfassend ein C-terminales Neoepitop, das durch Spaltung eines Titinproteins durch eine Protease in die Teilsequenz des Proteins gemäß Anspruch 1 gebildet wird, codiert.

9. Vektor, umfassend eine Nukleinsäuresequenz, umfassend ein Expressionssignal und eine codierende Sequenz, die für die Expression eines Peptids, umfassend ein C-terminales Neoepitop, das durch Spaltung eines Titinproteins durch eine Protease in die Teilsequenz des Proteins gemäß Anspruch 1 gebildet wird, codiert, oder Wirtszelle, die mit einem derartigen Vektor transformiert ist und ein derartiges Peptid exprimiert.

10. Immuntestkit, umfassend einen immunologischen Bindungspartner nach Anspruch 5 und ein konkurrierendes Mittel, das den immunologischen Bindungspartner bindet, und gegebenenfalls eines oder mehrere aus einem Waschreagens, einem Puffer, einem Stoppreagens, einer Enzymmarkierung, einem Enzymmarkierungssubstrat, Kalibrierungsstandards, einem Anti-Maus-Antikörper und Anweisungen zum Durchführen eines Tests unter Verwendung des Kits.

## Revendications

1. Procédé de dosage biologique permettant la quantification de fragments peptidiques comprenant un néo-épitope formé par clivage d'une protéine titine au niveau d'un site de clivage par une protéinase, ledit procédé consistant à mettre en contact un échantillon comportant lesdits fragments peptidiques avec un partenaire de liaison immunologique ayant une affinité de liaison spécifique pour ledit néo-épitope et à déterminer le niveau de liaison dudit partenaire de liaison immunologique aux fragments peptidiques présents dans ledit échantillon, ledit partenaire de liaison immunologique ayant une affinité de liaison spécifique pour la séquence suivante au niveau de la terminaison C d'un peptide : **NVTVEARLIK↓ '13542 SEQ ID N° : 2,** ledit site de clivage par protéinase étant indiqué par ↓.

2. Procédé selon l'une quelconque des revendications précédentes, ledit procédé étant effectué sous la forme d'un dosage immunologique compétitif dans lequel ledit partenaire de liaison immunologique et un agent de compétition sont incubés en présence dudit échantillon et l'agent de compétition rivalise avec les fragments peptidiques présents dans l'échantillon pour se lier au partenaire de liaison immunologique.

3. Procédé selon l'une quelconque des revendications précédentes, ledit échantillon étant un échantillon d'urine, de sérum, de sang, de plasma ou de salive.

4. Procédé selon l'une quelconque des revendications précédentes, ledit échantillon étant un échantillon provenant d'un patient, ledit procédé consistant en outre à comparer le niveau déterminé de ladite liaison desdits fragments peptidiques à des valeurs caractéristiques (a) d'individus en bonne santé comparables et/ou (b) d'un état cardiaque pathologique.

5. Partenaire de liaison immunologique contre un néo-épitope C-terminal formé par clivage par protéinase d'une protéine titine, qui est spécifiquement immunoréactif avec la terminaison C de la séquence d'acides aminés : **NVTVEARLIK↓ '13542 SEQ ID N° : 2,** ledit site de clivage par protéinase étant indiqué par ↓.

6. Lignée cellulaire produisant un anticorps monoclonal qui est un partenaire de liaison immunologique selon la revendication 5.

7. Peptide comprenant un néo-épitope C-terminal formé par clivage d'une protéine titine par une protéase dans la séquence partielle de ladite protéine décrite dans la revendication 1.

8. Molécule d'acide nucléique isolée codant un peptide comprenant un néo-épitope C-terminal formé par clivage d'une protéine titine par une protéase dans la séquence partielle de ladite protéine décrite dans la revendication 1.

9. Vecteur comprenant une séquence d'acides nucléiques comportant un signal d'expression et une séquence codante qui code l'expression d'un peptide comprenant un néo-épitope C-terminal formé par clivage d'une protéine titine par une protéase dans la séquence partielle de ladite protéine décrite dans la revendication 1 ou cellule hôte transformée avec un tel vecteur et exprimant ledit peptide.

10. Kit de dosage immunologique comprenant un partenaire de liaison immunologique selon la revendication 5, et un agent de compétition qui se lie audit partenaire de liaison immunologique, et éventuellement un ou plusieurs éléments parmi un réactif de lavage, un tampon, un réactif d'arrêt, un marqueur enzymatique, un support de marqueur enzymatique, des étalons d'étalonnage, un anticorps anti-souris et des instructions permettant d'effectuer un dosage au moyen dudit kit.
